# EUROPEAN PATENT SPECIFICATION

(11) **EP 2 641 967 B1**
(45) Date of publication and mention of the grant of the patent: **12.10.2016**
(21) Application number: 11840973.9
(22) Date of filing: 17.11.2011
(51) Int. Cl.: C12N 5/10, C12N 5/0781, C12N 15/09, C40B 50/06

(54) **METHOD FOR PREPARING B CELL WHICH PRODUCES HUMAN-TYPE ANTIBODY**
VERFAHREN ZUR HERSTELLUNG EINER B-ZELLE DIE HUMANE ANTIKÖRPER PRODUZIERT
PROCÉDÉ POUR LA PRODUCTION D' UNE CELLULE-B QUI PRODUIT DES ANTICORPS DE TYPE HUMAIN

(30) Priority: 18.11.2010 JP 2010258404
(43) Date of publication of application: 25.09.2013
(73) Proprietor: National University Corporation Okayama University, Kita-ku Okayama-shi Okayama 700-8530 (JP)
(72) Inventor: KANAYAMA, Naoki, Okayama-shi Okayama 700-8530 (JP); OHMORI, Hitoshi, Okayama-shi Okayama 700-8530 (JP)
(74) Representative: Grünecker Patent- und Rechtsanwälte PartG mbB
(86) International application number: PCT/JP2011/076533
(87) International publication number: WO 2012/067188

(56) References cited:
- WO-A1-2007/026661
- WO-A1-2011/061937
- JP-A- 2006 109 711
- JP-A- 2010 528 626
- US-A- 5 202 238
- TODO K ET AL: "Novel in vitro screening system for monoclonal antibodies using hypermutating chicken B cell library", JOURNAL OF BIOSCIENCE AND BIOENGINEERING, ELSEVIER, AMSTERDAM, NL, vol. 102, no. 5, 1 November 2006 (2006-11-01), pages 478-481, XP028042364, ISSN: 1389-1723, DOI: 10.1263/JBB.102.478 [retrieved on 2006-11-01]
- OHMORI H ET AL: "Efficient in vitro antibody generation system using an engineered chicken B cell line, DT40-SW", JOURNAL OF BIOTECHNOLOGY, ELSEVIER SCIENCE PUBLISHERS, AMSTERDAM, NL, vol. 150, 1 November 2010 (2010-11-01), pages 440-441, XP027490111, ISSN: 0168-1656, DOI: 10.1016/J.JBIOTEC.2010.09.628 [retrieved on 2010-11-01]
- KAJITA M ET AL: "Efficient affinity maturation of antibodies in an engineered chicken B cell line DT40-SW by increasing point mutation", JOURNAL OF BIOSCIENCE AND BIOENGINEERING, ELSEVIER, AMSTERDAM, NL, vol. 110, no. 3, 1 September 2010 (2010-09-01), pages 351-358, XP027228231, ISSN: 1389-1723 [retrieved on 2010-04-11]
- KANAYAMA N ET AL: "Reversible switching of immunoglobulin hypermutation machinery in a chicken B cell line", BIOCHEMICAL AND BIOPHYSICAL RESEARCH COMMUNICATIONS, ACADEMIC PRESS INC. ORLANDO, FL, US, vol. 327, no. 1, 4 February 2005 (2005-02-04), pages 70-75, XP004697896, ISSN: 0006-291X, DOI: 10.1016/J.BBRC.2004.11.143
- N. KANAYAMA ET AL: "Affinity maturation of a mouse monoclonal antibody in an in vitro antibody generation system using the hypermutating chicken B cell line", INTERNATIONAL IMMUNOLOGY MEETING ABSTRACTS :. DOI: 10.1093/INTIMM/DXQ243 THIS ARTICLE APPEARS IN:DAY 3: 14TH INTERNATIONAL CONGRESS OF IMMUNOLOGY KOBE, JAPAN ABSTACT PP-063-28, vol. 22, no. (Suppl 1 Pt 3), 25 August 2010 (2010-08-25), pages iii125-iii131, XP55108514,
- ZOU Y-R ET AL: "Cre-loxP-mediated gene replacement: a mouse strain producing humanized antibodies", CURRENT BIOLOGY, CURRENT SCIENCE, GB, vol. 4, no. 12, 1 December 1994 (1994-12-01), pages 1099-1103, XP024248918, ISSN: 0960-9822, DOI: 10.1016/S0960-9822(00)00248-7 [retrieved on 1994-12-01]
- DANIELIAN P S ET AL: "MODIFICATION OF GENE ACTIVITY IN MOUSE EMBRYOS IN UTERO BY A TAMOXIFEN-INDUCIBLE FORM OF CRE RECOMBINASE", CURRENT BIOLOGY, CURRENT SCIENCE, GB, vol. 8, no. 24, 23 November 1998 (1998-11-23), pages 1323-1326, XP000993084, ISSN: 0960-9822, DOI: 10.1016/S0960-9822(07)00562-3
- NAOKI KANAYAMA ET AL.: 'Creation of Valuable Antibodies by an In Vitro Antibody Generation System Using a Hypermutating B Cell Line' JOURNAL OF THE PHARMACEUTICAL SOCIETY OF JAPAN vol. 129, no. 1, 2009, pages 11 - 17, XP055093954
- NAOKI KANAYAMA: 'Basic Research and Development on Generation Mechanism of High Affinity Antibodies' JOURNAL OF THE SOCIETY FOR BIOSCIENCE AND BIOENGINEERING vol. 87, no. 3, 2009, JAPAN, pages 116 - 122, XP008168467
- NAOKI KANAYAMA ET AL.: 'Hitogataka Chimera Kotai o Hatsugen suru Niwatori B Saibokabu no Juritsu to Oyo' BMB2010 KOEN YOSHISHU 19 November 2010, page 1P-1267, XP008168470
- PLUSCHKE G. ET AL.: 'Generation of chimeric monoclonal antibodies from mice that carry human immunoglobulin Cyl heavy or CK light chain gene segments' J. IMMUNOL. METHODS vol. 215, no. 1-2, 1998, pages 27 - 37, XP004146490
- 'Biotechnology Iryo Gijutsu Kaihatsubu, Heisei 21 Nendo Jissihoshin', [Online] 30 November 2011, XP055108565 Retrieved from the Internet: <URL:http://www.nedo.go.jp/content/10008380 2.pdf>
- 'Biotechnology Iryogijutsu Kaihatsubu, Heisei 22 Nendo Jissihoshin', [Online] 30 November 2011, XP055108566 Retrieved from the Internet: <URL:http://www.nedo.go.jp/content/10008380 3.pdf>

### Sequence Listing

### SEQUENCE LISTING

<110> NATIONAL UNIVERSITY CORPORATION OKAYAMA UNIVERSITY
<120> A method of producing B cell which priduces humanized antibody
<130> PH-5017-PCT
<150> JP 2010-258404
   <151> 2010-11-18
<160> 38
<170> PatentIn version 3.4
<210> 1
   <211> 65
   <212> DNA
   <213> Artificial
<220>
   <223> synthetic
<400> 1
   acaacagtgg tgctgcattt ggggccggga caacctgacc gtcctaggaa ctgtggctgc 60
   accat 65
<210> 2
   <211> 158
   <212> DNA
   <213> Artificial
<220>
   <223> synthetic
<400> 2
   atctcctcca ttggcctctg gaggccgaag aggacgacga catccaacgc ctttgggcca 60
   ccacctccac cttcatcgtc ctcttcatcc tcagcctctt ctacagcgcc accgtcaccc 120
   tcatcaaggt gaaatgaccc caaatttcca tccggatt 158
<210> 3
   <211> 2138
   <212> DNA
   <213> Artificial
<220>
   <223> synthetic
<400> 3
   tggatagggc ttcgggtaaa gcaagtgctg tcaatgtctc cttggtgttg gccgactcgg 60
   ccgccgcctg ctattaatta attaacccgc tcgttaagcg gccgctcgat tgggattaaa 120
   gagcagatgt ccattggacg gggttttggg gtcatcgatt tcattcgggg gtcatcacct 180
   caattgagac ggtttggggt cattgacaac atgggtaggg agtggattca taattagacg 240
   gggtctgtta attaggataa tgagagcgat tagggcttca ttgggctcta ttgggtcgtt 300
   aatgactggg agggattggg tggcgtcggt tccaaggagg tgtttggggt catcgttggg 360
   atatgttggg tcaatgtcaa gaaagggata gactggggtc attagtgtaa ttagagggct 420
   taattggggc gtcgtcttaa ttagggtcat cggagtcatc gtcttaatta gagggattgg 480
   gggacgtagg ttcaataggg agagtttggg gtcatcgatg gatacagctg gagttgaggt 540
   cactatcaga cgtgtttggg gtcattagtg taattagagg gcttaattgg gccatcatct 600
   tcattaggat cgtcagactc atcgtcttaa ttagagggat tggggttcat aggttcaata 660
   gggagagttt gtggtcatcg atgggtacag ctggagttgg ggtcactatc acacgagttt 720
   ggggtcatta gtgcaattag agggcttaat tgggccatca tcttcattag gatcatcgga 780
   gtcatcgtct taattagagg gattggggtt catgggttta attgggtgag tttggggtca 840
   ttattgtctt aattgggaga gtttggggtc atcgatggga gagttgggat cagcatcact 900
   atcagaagcg attggggtca ttagtgtaat tagagggctt aattgggcca tcatcgtaat 960
   taggatcgtc agactcatcg tcttaattag agggattggg gttcataggt ttaattggga 1020
   gagtttgagg tcatcatcgt cttaattggg agagtttggg gtcatcgatg ggagagttgg 1080
   gatcagcgtc actatcagaa gagattgggg tcattagtgt aattagaggg cttaattggg 1140
   cccaacgtct taattaggat cattggagtc atcgtcttaa ttagagggat tggggttcat 1200
   gggattaatt gggagagttt gcggtcatta tcttaattcg gagaggtcgg ggtgggtgtc 1260
   atcgtttggg gagtgggggt cattaacgta attaaagcca ttaattgggt cattatctta 1320
   attacgatca tcggagtcac cgtcttcatt agcgggattg gggtttgtgg gtttaattgg 1380
   aggagtttgg ggtcattatc ttaattagga gagtttaatt aggggtgggc gtcatcgttg 1440
   ggggattggg gatcattaac gtaattagag ccatcaattg ggtcattatc ttaattagga 1500
   tcatcagagt caccgtctta attagaggga ttggggttca tgggtttaat tggggtttaa 1560
   ttgggtctta attgcaatcg ggagcgtttg gggtcatcgt tctcattggg agacttcggg 1620
   tcagtgccac cattgggtca ttaattactt ggggttaatt agcgtctaat tagaagggtt 1680
   tgggtcatct taattagagg gattggggtt tgtgggttta attgggagag tttggggtca 1740
   ttatcttaat taggagagtt taattagggg tgggcgtcat cgttggggga ttggaggtca 1800
   ttaacgtaat taaagccatt aattgggtca ttatcttaat taggatcatt ggagtcatgg 1860
   tcttaattag agggattggg gttcgtgggt ttaattgggg ttaattgggt cttaattgca 1920
   atcgggagcg tttggggtca tcgttctcat tgggagactt cgggtcagtg ccaccattgg 1980
   gtcattaatt acttggggtt aattagcgtc taattagaag ggtttgggtc catttccccc 2040
   ccaaaatccc catttccgac cccaaaatcc ccatttccga ccccaaaatc cccatttccg 2100
   accccaaaat ctcccatttc ccccccaaaa tccccatt 2138
<210> 4
   <211> 297
   <212> DNA
   <213> Artificial
<220>
   <223> synthetic
<400> 4
   ccccatttcc cccccaaaat ccccatttcc gaccccaaaa tccccatttc cgaccccgaa 60
   aatccccatt tccgaccccc aaatcccatt tctgaccccc aaatccccca tttcccccca 120
   atttccccca tccccccccc acaccccctc gccgaataca aaacagaccc aaaatcgccc 180
   gaatttaccc caaaacggcg tcggtttttc ttttgcctca gatctcctcc attggcctct 240
   ggaggccgaa gaggacgacg acatccaacg cctttgggcc accacctcca ccttcat 297
<210> 5
   <211> 38
   <212> DNA
   <213> Artificial
<220>
   <223> primer
<400> 5
   gagtcgctga actagtctcg gtctttcttc ccccatcg 38
<210> 6
   <211> 40
   <212> DNA
   <213> Artificial
<220>
   <223> primer
<400> 6
   acggatccat atctattttc atggatgtta tacgtgtgcg 40
<210> 7
   <211> 36
   <212> DNA
   <213> Artificial
<220>
   <223> primer
<400> 7
   ctaagcttcc cactggggat gcaatgtgag gacagt 36
<210> 8
   <211> 39
   <212> DNA
   <213> Artificial
<220>
   <223> primer
<400> 8
   tgcgatccag gtaccacgat agcactgcct gcctccatc 39
<210> 9
   <211> 40
   <212> DNA
   <213> Artificial
<220>
   <223> primer
<400> 9
   ctaaactctg aggggatccg atgacgtggc cattctttgc 40
<210> 10
   <211> 40
   <212> DNA
   <213> Artificial
<220>
   <223> primer
<400> 10
   gtaagcttct aacactctcc cctgttgaag ctctttgtga 40
<210> 11
   <211> 35
   <212> DNA
   <213> Artificial
<220>
   <223> primer
<400> 11
   ccatcggcgt ggggacacac agctgctggg attcc 35
<210> 12
   <211> 43
   <212> DNA
   <213> Artificial
<220>
   <223> primer
<400> 12
   gtgatgatga ggctactgct gactctcaac attctactcc tcc 43
<210> 13
   <211> 39
   <212> DNA
   <213> Artificial
<220>
   <223> primer
<400> 13
   gagaggccaa agtacagtgg aaggtggata acgccctcc 39
<210> 14
   <211> 40
   <212> DNA
   <213> Artificial
<220>
   <223> primer
<400> 14
   ggagctgtac catgcggcct gctctgctga tgccatgtcg 40
<210> 15
   <211> 30
   <212> DNA
   <213> Artificial
<220>
   <223> primer
<400> 15
   cggcgtgggg atccacagct gctgggattc 30
<210> 16
   <211> 24
   <212> DNA
   <213> Artificial
<220>
   <223> primer
<400> 16
   ggagccatcg atcacccaat ccac 24
<210> 17
   <211> 40
   <212> DNA
   <213> Artificial
<220>
   <223> primer
<400> 17
   ctcatcagat ggcgggaaga tgaagacaga tggtgcagcc 40
<210> 18
   <211> 30
   <212> DNA
   <213> Artificial
<220>
   <223> primer
<400> 18
   ctgactgacc gcgttactcc cacagccagc 30
<210> 19
   <211> 30
   <212> DNA
   <213> Artificial
<220>
   <223> primer
<400> 19
   ttcatgaggt agtccgtcag gtcacggcca 30
<210> 20
   <211> 40
   <212> DNA
   <213> Artificial
<220>
   <223> primer
<400> 20
   aaatggccga attgagctcg gccgttttac ggttgggttc 40
<210> 21
   <211> 40
   <212> DNA
   <213> Artificial
<220>
   <223> primer
<400> 21
   ctcatcattc agtatcgatg gatccttaat tactcccacg 40
<210> 22
   <211> 27
   <212> DNA
   <213> Artificial
<220>
   <223> primer
<400> 22
   ggctcagcgt cacctgcatg gctcagg 27
<210> 23
   <211> 39
   <212> DNA
   <213> Artificial
<220>
   <223> primer
<400> 23
   ccttgatttc gaagtggaga agacgtcggg aggtggaga 39
<210> 24
   <211> 40
   <212> DNA
   <213> Artificial
<220>
   <223> primer
<400> 24
   tggatagggc ttcgggtaaa gcaagtgctg tcaatgtctc 40
<210> 25
   <211> 39
   <212> DNA
   <213> Artificial
<220>
   <223> primer
<400> 25
   atgaaggtgg aggtggtggc ccaaaggcgt tggatgtcg 39
<210> 26
   <211> 40
   <212> DNA
   <213> Artificial
<220>
   <223> primer
<400> 26
   acggatcctg caagctttct ggggcaggcc aggcctgacc 40
<210> 27
   <211> 40
   <212> DNA
   <213> Artificial
<220>
   <223> primer
<400> 27
   cggcggccgc actcatttac ccggagacag ggagaggctc 40
<210> 28
   <211> 40
   <212> DNA
   <213> Artificial
<220>
   <223> primer
<400> 28
   taatcggtac tttttaatcc tccattttgc ccgaaatcgc 40
<210> 29
   <211> 37
   <212> DNA
   <213> Artificial
<220>
   <223> primer
<400> 29
   ctgtggtgtg acataattgg acaaactacc tacagag 37
<210> 30
   <211> 32
   <212> DNA
   <213> Artificial
<220>
   <223> primer
<400> 30
   tggactccga cggctccttc ttcctctaca gc 32
<210> 31
   <211> 38
   <212> DNA
   <213> Artificial
<220>
   <223> primer
<400> 31
   ccttgatgag ggtgacggtg gcgctgtaga agaggctg 38
<210> 32
   <211> 30
   <212> DNA
   <213> Artificial
<220>
   <223> primer
<400> 32
   gccggaattc cgacggagga gcaccagtcg 30
<210> 33
   <211> 29
   <212> DNA
   <213> Artificial
<220>
   <223> primer
<400> 33
   ttgtaccacg tgacctcggt gggacggcg 29
<210> 34
   <211> 30
   <212> DNA
   <213> Artificial
<220>
   <223> primer
<400> 34
   ctcttggagg agggtgccag ggggaagacc 30
<210> 35
   <211> 22
   <212> DNA
   <213> Artificial
<220>
   <223> primer
<400> 35
   ggcggctccg tcagcgctct ct 22
<210> 36
   <211> 20
   <212> DNA
   <213> Artificial
<220>
   <223> primer
<400> 36
   gccgcaaatg atggaccgac 20
<210> 37
   <211> 25
   <212> DNA
   <213> Artificial
<220>
   <223> primer
<400> 37
   ggcacggagc tctgtcccat tgctg 25
<210> 38
   <211> 24
   <212> DNA
   <213> Artificial
<220>
   <223> primer
<400> 38
   ccccagcctg ccgccaagtc caag 24

## Description

### Technical Field

The present invention relates to a method for preparing B cells which produce a human-type antibody.

### Background Art

Antibodies that are produced *in vivo* by immune system are increasingly being applied not only to reagents, but also currently to medicines and diagnostic agents, making use of their capacity to recognize specific targets. *In vivo*, the affinity of an antibody that is produced in response to antigen stimulation is increased over time. This is referred to as affinity maturation and it proceeds as follows. In antigen specific B cells activated to actively divide, high-frequency somatic mutation takes place in antibody variable region genes, and then from the resulting various mutant B cell populations, B cell clones having acquired high affinity are strictly selected. With the use of this principle, repeated immunization of animals and monoclonal antibody preparation by hybridoma preparation are broadly carried out. However, these techniques are problematic in that much effort and time are required for obtaining antibodies. For example, antibodies against antigens with high inter-species conservation are obtained with difficulty because of immunologic tolerance, and obtainment of bioactive antibodies is also said to be difficult. These antibodies, the preparation of which using animals is difficult, are often useful for medicines. Hence, a phage display method that is an *in vitro* technique not involving immunologic tolerance is currently in frequent use. With the phage display method, antibody selection can be rapidly carried out compared with a hybridoma method, but this is known to be problematic in that: the success or failure of antibody preparation significantly depends on library quality; and since an antibody Fv fragment is recombined as scFv and then displayed on a phage, the specificity is often altered when expressed in complete antibody form. In particular, mutant library construction as a key procedure requires much effort and advanced gene recombination techniques.

Accordingly, if an *in vivo* antibody production system can be reproduced with an *in vitro* cultured cell system, an antibody can be prepared rapidly and efficiently. A DT40 chicken B cell line retaining the capacity to introduce a mutation into an antibody gene is appropriate for achieving the purpose in respect of points mentioned bellow.

(1) Various antibody libraries, which are not affected by immunologic tolerance, are constructed by culture alone using spontaneous mutagenesis capacity.

(2) An antibody is expressed on a cell surface and in a culture supernatant, and then a specific clone can be selected based on binding to an antigen.

(3) Because of very high homologous recombination efficiency, cell functions can be easily altered by gene knock out or the like.

The present inventors have focused on the properties of DT40, established a DT40-SW cell line capable of turning ON/OFF the mutation functions of DT40 arbitrarily, and thus developed an *in vitro* method for preparing an antibody using DT40-SW (see Patent Document 1 and Non-patent Document 1) (Fig. 1). With this method, antibodies (including those difficult to be obtained by a conventional method) against various antigens have been successfully obtained from antibody libraries obtained by turning mutation functions ON followed by culturing (see Non-patent Documents 2 and 3). Moreover, with the method, a mutation is introduced again into the thus obtained antibody-producing cells for diversification and then selected. These procedures are repeated so that antibody affinity maturation is possible. In particular, more efficient affinity maturation has also been achieved with the method through manipulation of the pattern of mutagenesis (see Patent Document 3 and Non-patent Documents 4 and 5).

For application of an antibody to a medicine, the use of a human antibody or a humanized antibody is essential. This is because: when a human ingests a heterologous antibody (antibody derived from a different species), an immune reaction takes place *in vivo* against the ingested antibody, which is foreign matter in the human body; and repeated dose causes problems such that a severe adverse reaction is induced or the effects become attenuated. Therefore, antibody drug development requires humanization of a candidate heterologous antibody at the initial evaluation stage, or obtainment of an antibody having a human-type structure as a candidate antibody. In particular, for evaluation of the effects by *in vitro* or *in vivo* tests, an antibody constant region is essentially of a human IgG1 type. Examples of a conventionally employed method for obtaining a human antibody or a humanized antibody include (a) a method that involves obtaining a chimera of an antibody variable region of a heterologous antibody and a human antibody constant region by a hybridoma method or the like and then expressing it in host cells (see Patent Document 3), (b) CDR transplantation that involves transplanting an antigen binding portion of a variable region to a human antibody (see Patent Documents 4 and 5), (c) a method that involves obtaining an antibody from a phage display library displaying a human antibody fragment prepared from an antibody gene group isolated from a human (see Patent Documents 6 and 7), and (d) a method that involves obtaining an antibody by a hybridoma method from a mouse into which a human antibody gene has been introduced (see Patent Documents 8 to 10).

### Prior Art Documents

Patent Documents

Patent Document 1: JP Patent No. 4487068
Patent Document 2: JP Patent Publication (Kokai) No. 2009-60850 A
Patent Document 3: U.S. Patent No. 6331415
Patent Document 4: U.S. Patent No. 5225539
Patent Document 5: U.S. Patent No. 5585089
Patent Document 6: U.S. Patent No. 5885793
Patent Document 7: U.S. Patent No. 5837500
Patent Document 8: U.S. Patent No. 6150584
Patent Document 9: U.S. Patent No. 5545806
Patent Document 10: JP Patent No. 3030092

### Non-patent Documents

Non-patent Document 1: Kanayama, N., et al. Biochem. Biophys. Res. Commun. 327, 70-75
Non-patent Document 2: Todo, K., et al. J. Biosci. Bioeng. 102, 478-481 (2006)
Non-patent Document 3: Kanayama, N., et al. YAKUGAKU ZASSHI 129, 11-17 (2009)
Non-patent Document 4: Kajita, M., et al. J. Biosci. Bioeng. 110, 351-358 (2010)
Non-patent Document 5: Kajita, M., J. Biosci. Bioeng. 109, 407-410 (2010)

### Summary of the Invention

### Problem to be Solved by the Invention

An object of the present invention is to provide a method for preparing B cells that produce a human-type antibody and are capable of performing spontaneous mutagenesis.

### Means for Solving the Problem

The present inventors have considered that since an antibody obtained from the above DT40-SW is a chicken IgM antibody, if an antibody preparation system using DT40-SW enables obtainment of a human IgG1 antibody, this will be a technique very useful for pharmaceutical development.

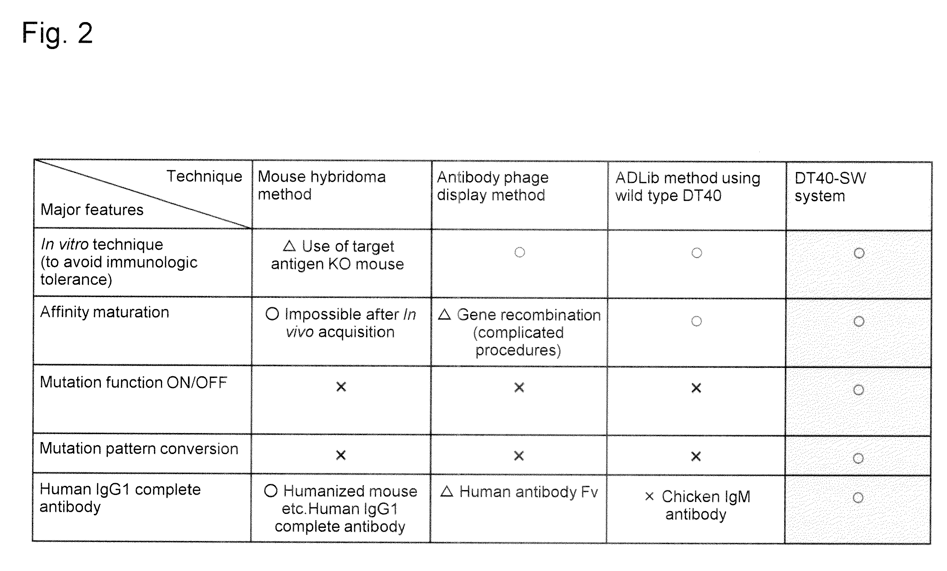

As described above, antibody search for medical applications requires preparation of a human antibody or a humanized antibody. The above techniques (a) to (d) for obtaining a human antibody and a humanized antibody are broadly employed, but are potentially problematic as follows. For example, (a), (b), and (d) are problematic in terms of immunologic tolerance since preparation of a candidate antibody is based on a hybridoma method that depends on immunization of mice, (c), which uses a phage display, requires the alteration of the obtained antibody to result in a complete antibody, and (a), (b), and (c) require many genetic recombination techniques and thus are complicated in cases in which many candidate antibodies are handled (Fig. 2).

Moreover, the affinity and the specificity of a once obtained antibody cannot be easily improved by conventional techniques other than the DT40-SW system. A cell line having mutation capacity such as DT40 has been used as a library after introduction of a mutation into cells' original antibody gene (Cumbers, S. J., et al. Nat. Biotechnol. 20, 1129-1134 (2002); Seo, H., et al. Nat. Biotechnol. 23, 731-735 (2005); Todo, K., et al. J. Biosci. Bioeng. 102, 478-481 (2006)). Antibody preparation using DT40 can be performed without the problem of immunologic tolerance. However, the thus obtained antibodies are all chicken IgM antibodies, and thus application of the above techniques (a) and (b) is required under current circumstances. When the DT40-SW system is compared with other conventional techniques, many advantages are found with the DT40 system (Fig. 2), and thus the development of an altered DT40-SW system that is able to produce a human antibody is clearly important.

The present inventors have considered that it is important for solving the above problems that an antibody preparation system is provided with the following characteristics (1) to (4).
(1) The antibody preparation system is an *in vitro* technique capable of avoiding immunologic tolerance.
(2) A mutation is efficiently introduced into an antibody variable region gene.
(3) An antibody gene is displayed and expressed on a cell surface.
(4) An antibody gene is expressed and the product is secreted in a medium supernatant.

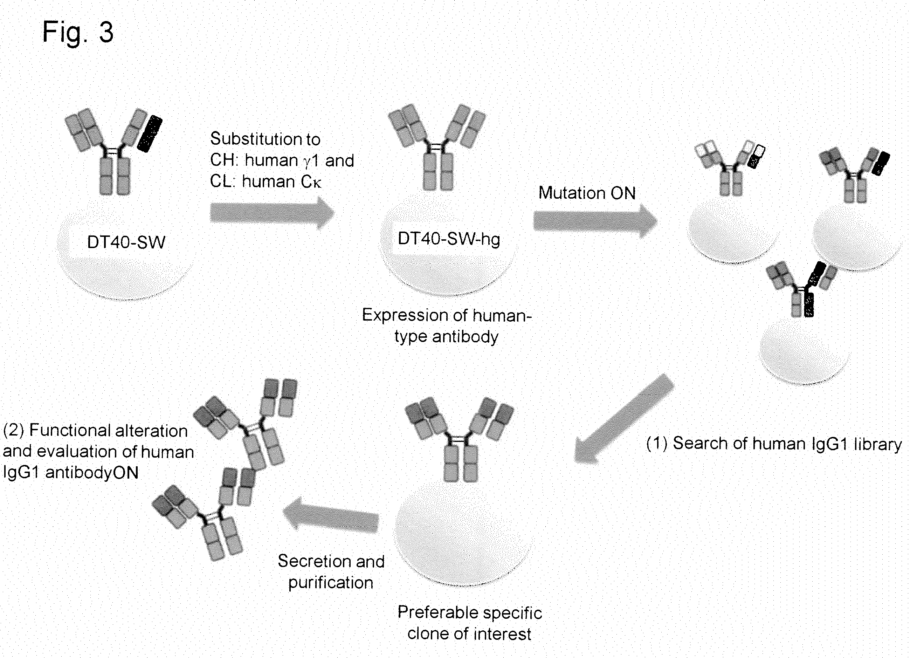

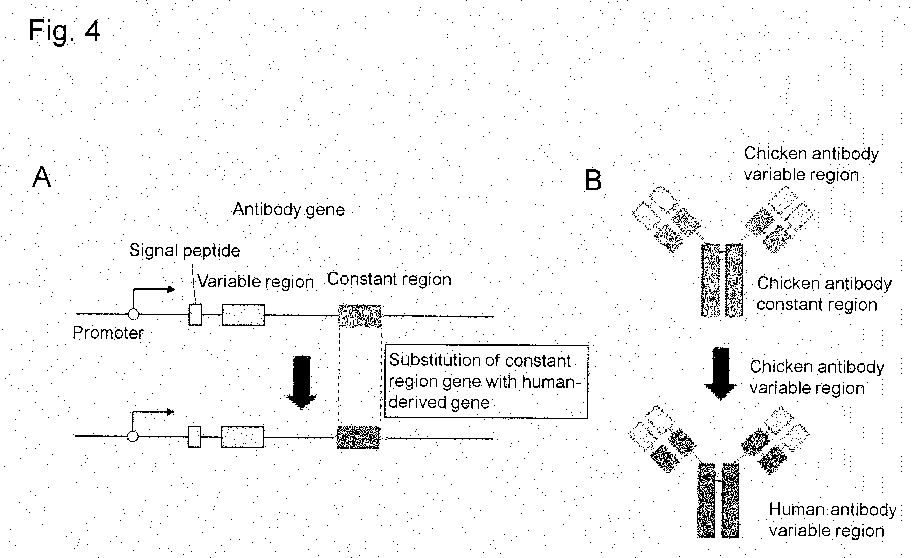

The above (1) and (2) are important for construction of an antibody library containing useful antibodies. The above (3) and (4) are important for screening for cells producing an antibody of interest. Accordingly, the present inventors have conceived of a method for converting DT40-SW cells to human-type antibody-producing cells by substituting a chicken antibody gene with a human antibody gene by high-frequency homologous recombination, one of the characteristics of DT40 cells (Fig. 3). Particularly, in the present invention, the present inventors have considered that substitution of a constant region with a human-type constant region while leaving a variable region, into which a mutation is introduced, is left as it is (chicken variable region) in order to construct a useful antibody library effectively utilizing the mutation capacity of DT40 cells (Fig. 4A). An antibody to be produced herein is a chicken-human chimeric antibody. Since the constant region thereof is derived from a human, and thus the antibody can be directly used for various tests for searching candidate medicines, enabling accelerated candidate search (Fig. 4B). A human-type antibody to be produced herein is preferably an IgG1 antibody that can be expected to exhibit various effector functions via *in vivo* heavy chain constant region. The present inventors have established herein human IgGI-producing DT40-SW and have used the κ chain, the amount of which existing *in vivo* in a human is the highest over the other parts, as a light chain constant region.

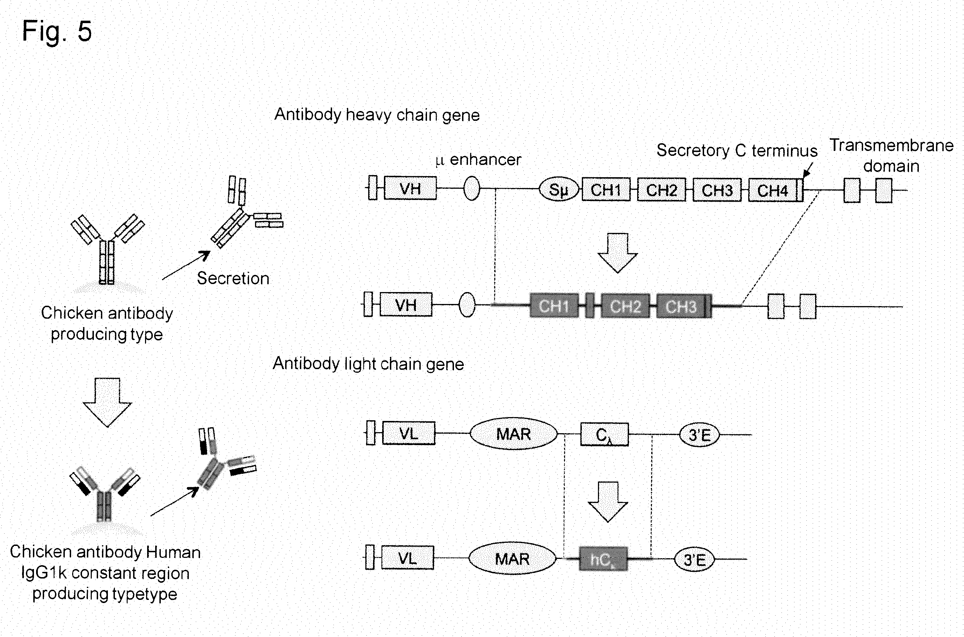

An antibody gene is composed of an exon (located downstream of a promoter) encoding a leader peptide that contains signals required for endoplasmic reticulum targeting, exons encoding a variable region, and exons encoding a constant region (Fig. 4A). In the case of a heavy chain, the constant region is composed of multiple exons, and whether the antibody is expressed in a secretory form or a membrane-bound form is determined by different exon types to be used. Therefore, the present inventors have concluded that the most efficient method for construction of cells provided with the above characteristics (2) to (4) comprises, for a heavy chain, substituting the region from CH1 to secretory exon (from among exons encoding the constant region) with a human-derived IgG1 antibody heavy chain constant region gene, and for a light chain, and substituting only the constant region exon with a human-derived κ light chain constant region gene (Fig. 5). At this time, since a region important for mutagenesis is thought to be present in an intron between a variable region gene and a constant region gene, the present inventors have considered that the substitution of the constant region without altering and/or eliminating the region is important.

Preparation of DT40-SW requires (i) isolation and structural analysis of a chicken antibody gene, and (ii) construction of a targeting vector for substitution of a constant region exon of the chicken antibody gene. The progress of genome analysis has revealed the nucleotide sequence of a light chain antibody gene region, but revealed only partial information of a heavy chain. The present inventors have isolated unknown regions and revealed the portions of the unknown sequences. Based on the thus revealed information, the present inventors have constructed a targeting vector for substitution of heavy chain and light chain antibody constant region genes and then succeeded in establishment of cells producing a chimeric antibody with the use of the vector. The cells expressed the chimeric antibody on the cell surfaces and in culture supernatants. The present inventors have attempted to introduce a mutation into an antibody variable region, succeeded in mutagenesis with efficiency equivalent to that of a case of wild-type DT40-SW in which a mutation is introduced into an endogenous antibody gene, and thus completed the present invention.

Specifically, the present invention is as follows.
[1] A method for preparing B cells which produce a human-type antibody, comprising substituting an antibody gene of B cells with a human antibody gene, wherein
   the B cells are non-human vertebrate B cells capable of inducing or halting AID (activation induced cytidine deaminase) expression with the induction of the expression of an exogenous Cre recombinase gene through extracellular stimulation followed by the inversion of the direction of the exogenous AID gene by expressed Cre recombinase, and
   the B cells are characterized in that:
   1) the endogenous AID gene is functionally disrupted, and an AID protein resulting from the expression of the endogenous AID gene is not produced;
   2) the B cells have an exogenous AID gene that is flanked by two loxP sequences in opposite directions to each other, and a promoter that is present upstream of the region flanked by the two loxP sequences and is capable of functioning in the animal cells,
      when the AID gene is placed in a forward direction with respect to the promoter, the AID gene can be expressed by the promoter, and
      when the AID gene is placed in a reverse direction with respect to the promoter, the expression of the AID gene is halted; and
   3) a Cre recombinase gene is introduced so that Cre recombinase activation by extracellular stimulation is possible, and the direction of the region flanked by the two loxP sequences, which contains the exogenous AID gene, is inverted by Cre recombinase activation.
[2] The method for preparing B cells which produce a human-type antibody according to [1], wherein the Cre recombinase gene of the non-human vertebrate B cells is present in a form such that a fusion protein with an estrogen receptor is expressed, and the extracellular stimulation is carried out by estrogen or a derivative thereof, such that cells are stimulated extracellularly by estrogen or a derivative thereof, so as to induce Cre recombinase activation intracellularly.
[3] The method for preparing B cells which produce a human-type antibody according to [1] or [2], wherein only the antibody gene constant region of a non-human vertebrate B cell is substituted with the human antibody gene constant region.
[4] The method for preparing B cells which produce a human-type antibody according to any one of [1] to [3], wherein in the case of the heavy chain, the region from a CH1 region to a secretory exon among exons encoding the constant region is substituted with a human-derived IgG antibody heavy chain constant region, and in the case of the light chain, only a constant region exon is substituted with a human-derived κ light chain constant region gene.
[5] The method for preparing B cells which produce a human-type antibody according to [1] to [4], wherein B cells producing a human-type antibody contain: a splicing receptor sequence and a splicing branch point sequence, which are derived from a human antibody gene, in an intron existing upstream of the constant region gene, a sequence of non-human vertebrate B cells as a poly A addition sequence existing downstream of the constant region gene; and a drug resistance gene flanked by loxP sequences located upstream of the human constant region gene containing a splicing sequence.
[6] The method for preparing B cells which produce a human-type antibody according to any one of [1] to [5], wherein the antibody gene of non-human vertebrate B cells is substituted with a human antibody gene using a targeting vector targeting the antibody gene of the B cells.
[7] The method for preparing B cells which produce a human-type antibody according to any one of [1] to [6], wherein the non-human vertebrate B cells are derived from a DT40 chicken B cell line.
[8] The method for preparing B cells which produce a human-type antibody according to [7], wherein the non-human vertebrate B cells are DT40-SW cells of the chicken B cell line.
[9] Non-human vertebrate-derived B cells producing a human-type antibody, which are obtained by the preparation method of any one of [1] to [8].
[10] A method for constructing a mutant human-type antibody library, comprising activating a Cre recombinase gene in the non-human vertebrate-derived B cells producing the human-type antibody of [9], culturing B cells, and then introducing a mutation into the antibody variable region gene.
[11] The method for constructing a mutant human-type antibody library according to [10], wherein Cre recombinase is present in non-human vertebrate-derived B cells producing a human-type antibody, such that a fusion protein with an estrogen receptor is expressed, Cre recombinase activation is induced intracellularly by stimulation of cells with estrogen or a derivative thereof extracellularly, and thus a mutation is introduced into the antibody variable region gene.

This description includes part or all of the content as disclosed in the descriptions and/or drawings of Japanese Patent Application No. 2010-258404, which is a priority document of the present application.

### Effects of the Invention

In the present invention, DT40-SW cells were improved to be able to display a human IgG1 antibody on cell surfaces and to secrete the same in a culture supernatant. The capacity of the DT40-SW-hg cell line to mutate an antibody variable region gene is better than DT40-SW that has already been used for preparation of antibodies against various antigens, making it possible to construct an even better antibody library. Furthermore, DT40-SW-hg possesses all useful characteristics of DT40-SW: (i) mutation and expression can be carried out within a cell in an integrated manner, so that a mutant antibody can be prepared conveniently, (ii) antibodies are displayed on cell surfaces, an antibody of interest can be easily selected based on its binding to an antigen, and the functions of an antibody of interest can be conveniently improved by mutation and selection, and (iii) mutation functions can be arbitrarily halted or restarted, and thus an antibody can be altered by fixation of a useful mutation or re-mutation. Moreover, DT40-SW-hg produces a human IgG1 antibody that can be easily handled as an antibody. An antibody Fc portion is of a human IgG1 type. Hence, the functions of an antibody, which must be evaluated when a useful antibody is searched for medicinal use (e.g., various immune reactions via the Fc portion such as antibody-dependent cell damage or complement-dependent cell damage), can be rapidly evaluated by directly using a complete human IgG1 antibody produced from a clone isolated from the antibody library of DT40-SW-hg. The antibody preparation system using the cell line prepared in the present invention can avoid various problems of conventional human-type antibody preparation methods, so that the system can be important basic technology for promoting the development of antibodies useful for treatment of intractable diseases such as cancer.

### Brief Description of the Drawings

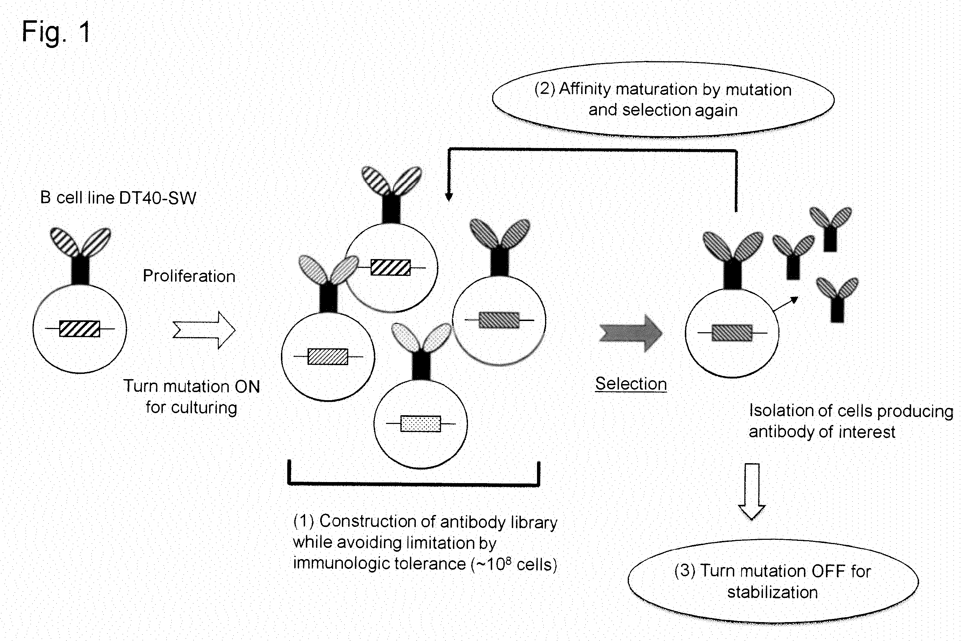

Fig. 1 shows the principle and usefulness of a DT40-SW system. Antibody-producing cells of interest are isolated from a DT40-SW library (1) and then mutation functions can be turned OFF for stabilization (3). A cycle of additional mutagenesis and selection is repeated, so that the affinity of the antibody can also be improved (2).
Fig. 2 shows a comparison of conventional methods with the technique for preparing an antibody of the present invention.
Fig. 3 shows methods for the functional alteration of a human IgG1 antibody in a human-type DT40-SW system and an antibody search of a human-type antibody library.
Fig. 4 shows the outline of a method for preparing human-type antibody-producing DT40. A chicken antibody constant region gene is substituted with a human antibody constant region gene by gene targeting to alter an antibody gene on the genome of DT40 cells (A), and then a chimeric antibody of the chicken antibody variable region and the human antibody constant region is expressed (B).
Fig. 5 shows the outline for preparation of human IgG1κ constant region-producing DT40.
Fig. 6 shows the outline of a method for substitution of a chicken antibody constant region gene. Fig. 6A shows the structure of a targeting vector, Fig. 6B shows the structure of the antibody gene locus of cells after targeting, Fig. 6C shows the structure of the antibody gene locus after treatment of cells (after targeting) with 4-hydroxytamoxifen followed by activation of Cre recombinase for elimination of a drug resistance gene, and Fig. 6D shows an example of a scheme for preparing human antibody constant region-producing DT40-SW cells.
Fig. 7 shows a method for introducing a human κ chain constant region gene targeting vector construct into DT40-SW. "A" denotes a chicken Cλ locus, "B" denotes a human Cκ targeting construct, and "C" denotes a human Cκ targeted locus. A diagram shown on the left in Fig. 7 is a schematic diagram showing sIg expression.
Fig. 8 shows the construction of a human κ chain constant region gene targeting vector.
Fig. 9 shows the results of confirming the introduction of the human κ chain constant region gene. Fig. 9A shows the results of selecting sIg-clone by FACS. Fig. 9B shows the results of confirming targeted locus by genomic PCR.
Fig. 10 shows the results of selecting clones expressing human κ chain at high levels. Fig. 10A shows the results of sorting (clone2) of hCκ-expressing cells, and specifically the results of single cell sorting of cells caused to recover sIg by 4-OHT treatment. Fig. 10B shows the results of selecting clones expressing hCκ at high levels by cell sorting.
Fig. 11 shows that DT40-SW-hk transcribes a chicken-human chimera light chain antibody gene. Fig. 11A shows the expression of a chimeric transcript of a chicken light chain variable region and a human κ chain constant region. Fig. 11B shows the result of the sequence analysis of an amplification product.
Fig. 12 shows the strategy for establishment of a human Cγ1 targeting construct and hIgG1-SW. Fig. 12A shows a chicken Cµ locus. Fig. 12B shows the human Cγ1 targeting construct. Fig. 12C shows the human Cγ1 targeted locus. A diagram on the left in Fig. 12 is a schematic diagram showing sIg expression.
Fig. 13 shows the construction of an hCγ1 targeting construct.
Fig. 14 shows the gene sequence of the transmembrane region of chicken membrane-type IgM.
Fig. 15-1 shows a 5' partial sequence of the IgH3' arm.
Fig. 15-2 shows a 3' partial sequence of the IgH3' arm.
Fig. 16 shows the results of confirming hCγ1 transfer. Fig. 16A shows the results of selecting the sIg-clone by FACS. Fig. 16B shows the results of confirming a targeted locus by genomic PCR.
Fig. 17 shows the results of selecting clones expressing hIgG1 at high levels. Fig. 17A shows the results of sorting hIgG1-expressing cells, wherein single cell sorting was performed for cells caused to recover sIg by 4-OHT treatment. Fig. 17B shows the results of selecting clones expressing hIgG1/hCκ at high levels by FACS. Fig. 17C shows the results of selecting clones with high antibody secretion levels by ELISA. Standard human IgG was subjected to serial dilution (5-320 ng/ml, Sample: 1-64 folds).
Fig. 18 shows that the proliferation capacity of DT40-SW-hg is equivalent to that of DT40-SW.
Fig. 19 shows that DT40-SW-hg transcribes a chicken-human chimeric IgG1 antibody gene. Fig. 19A shows L chain and Fig. 19B shows H chain.
Fig. 20 shows that DT40-SW-hg expresses a human IgG1 antibody. Fig. 20A shows the result for α-hIgG (×1: amount of protein: 50µg). Fig. 20B shows the result for α-hCκ (×1: amount of protein: 150 µg). Fig. 20C shows the result for α-cIgM (×1: amount of protein: 150 µg).
Fig. 21 shows that DT40-SW-hg secretes the human IgG1 antibody. Fig. 21A shows the secretion of the hIgG1 antibody. Fig. 21B shows the association of L chain with H chain. Fig. 21C shows the secretion of the cIgM antibody.
Fig. 22 shows that DT40-SW-hg introduced a mutation into an antibody variable region with high frequency.
Fig. 23-1A shows that DT40-SW-hg introduced a mutation into an antibody variable region (heavy chain variable region) with high frequency (clone 1). Underlined parts indicate CDRs. The sequence of OFF, 1, 5, 6, 12, 18, 19, 20, 22, 24, 29, 30, 31 and 32 are corresponding to SEQ ID NOs: 39 to 52, respectively.

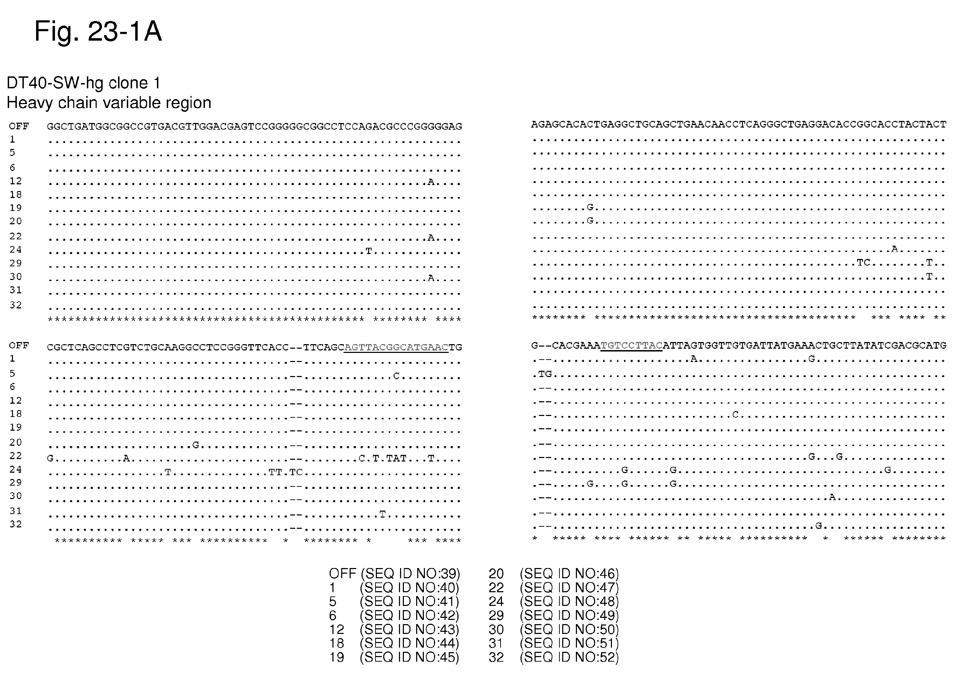

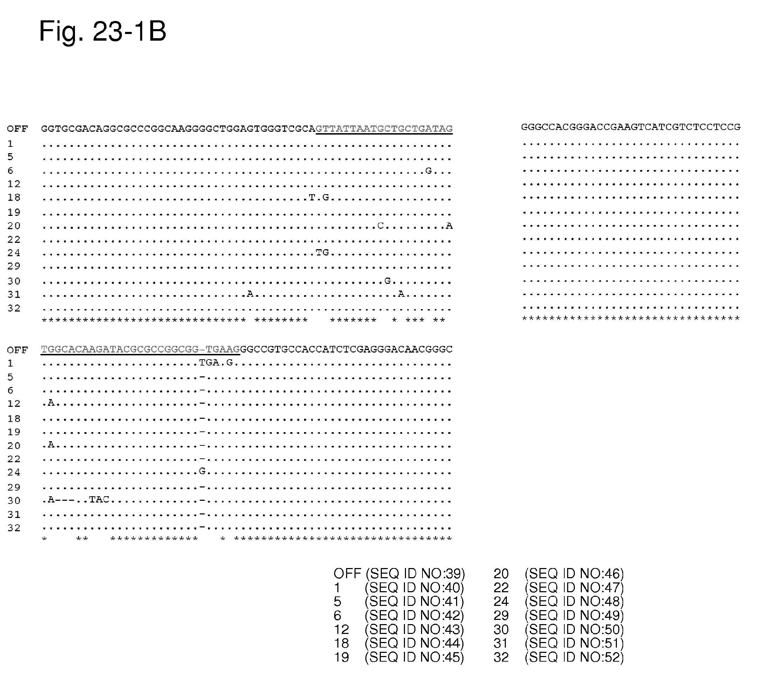

Fig. 23-1B shows that DT40-SW-hg introduced a mutation into an antibody variable region (heavy chain variable region) with high frequency (continuation of Fig. 23-1A). The sequence of OFF, 1, 5, 6, 12, 18, 19, 20, 22, 24, 29, 30, 31 and 32 are corresponding to SEQ ID NOs: 39 to 52, respectively.

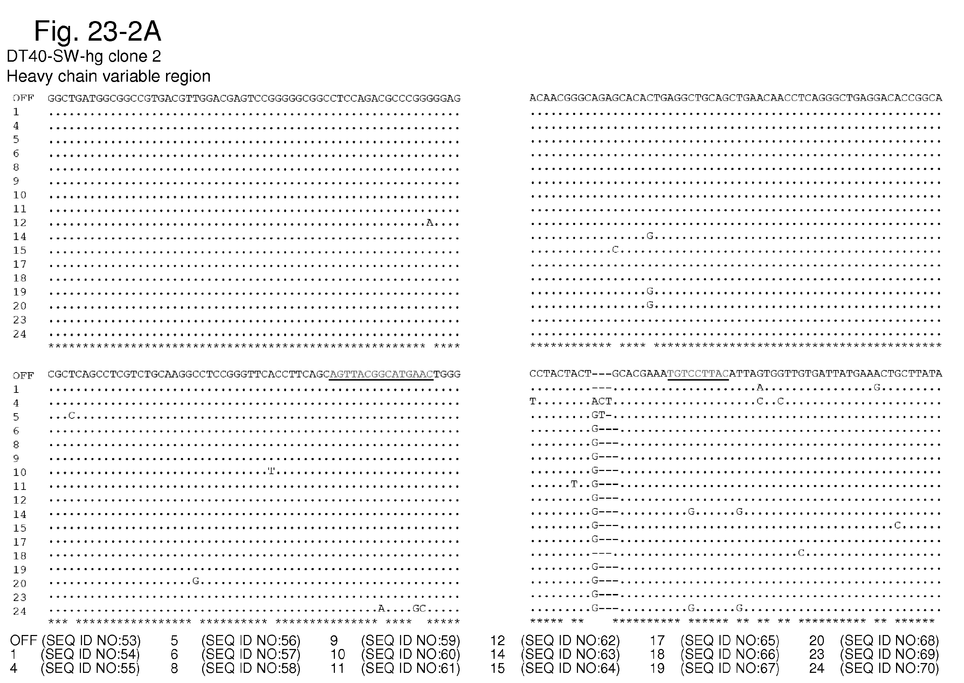

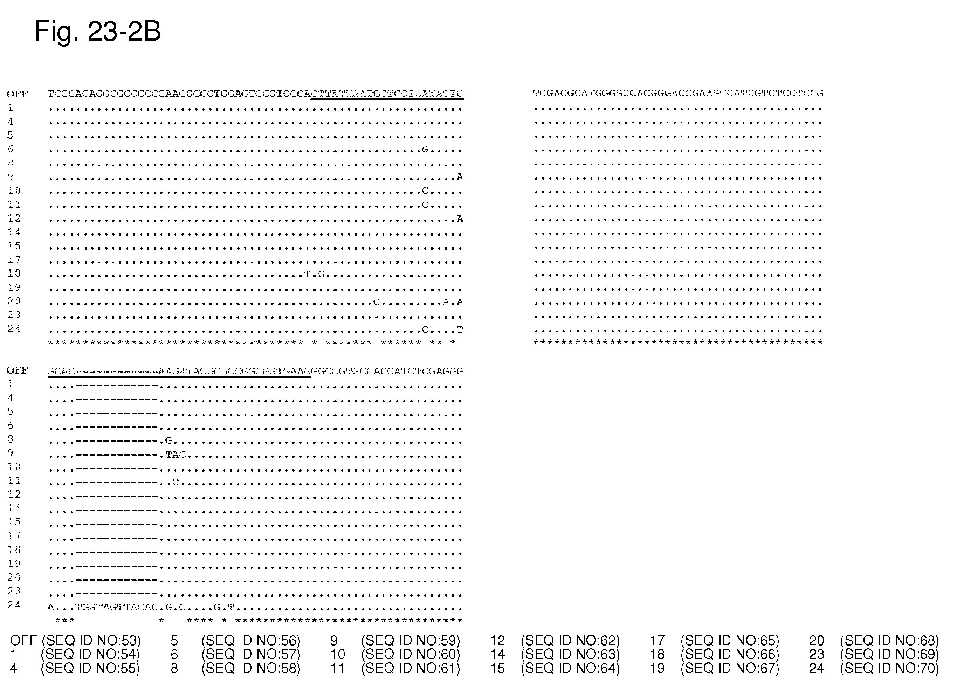

Fig. 23-2A shows that DT40-SW-hg introduced a mutation into an antibody variable region (heavy chain variable region) with high frequency (clone 2). Underlined parts indicate CDRs. The sequence of OFF, 1, 4, 5, 6, 8, 9, 10, 11, 12, 14, 15, 17, 18, 19, 20, 23 and 24 are corresponding to SEQ ID NOs: 53 to 70, respectively.
Fig. 23-2B shows that DT40-SW-hg introduced a mutation into an antibody variable region (heavy chain variable region) with high frequency (continuation of Fig. 23-2A). The sequence of OFF, 1, 4, 5, 6, 8, 9, 10, 11, 12, 14, 15, 17, 18, 19, 20, 23 and 24 are corresponding to SEQ ID NOs: 53 to 70, respectively.

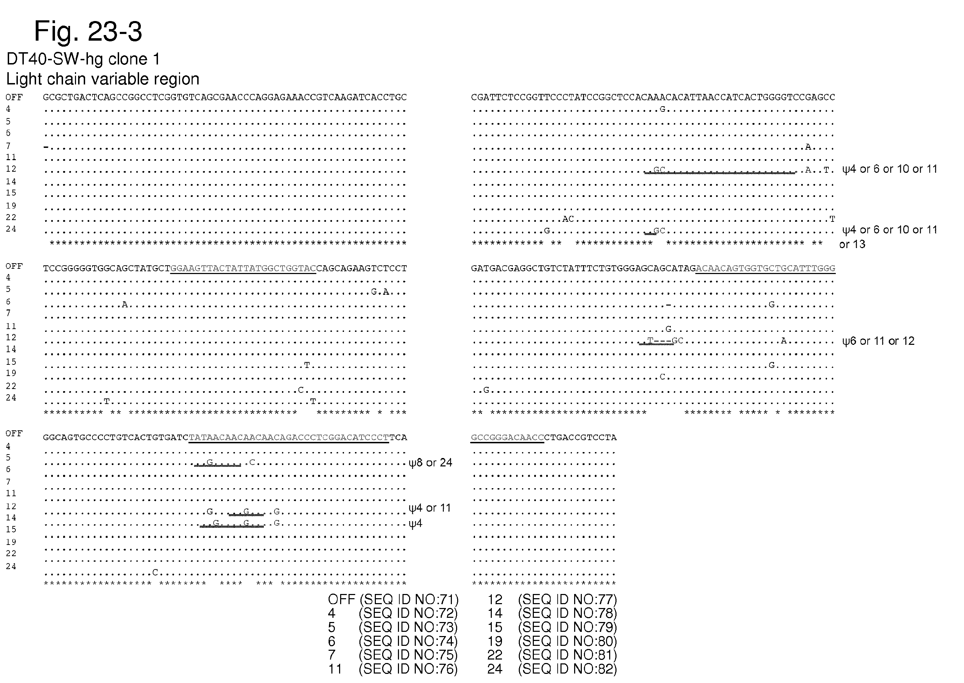

Fig. 23-3 shows that DT40-SW-hg introduced a mutation into an antibody variable region (light chain variable region) with high frequency (clone 1). Underlined parts indicate CDRs and genes described outside the columns are pseudo genes inferred to be used for gene conversion. The sequence of OFF, 4, 5, 6, 7, 11, 12, 14, 15, 19, 22 and 24 are corresponding to SEQ ID NOs: 71 to 82, respectively.

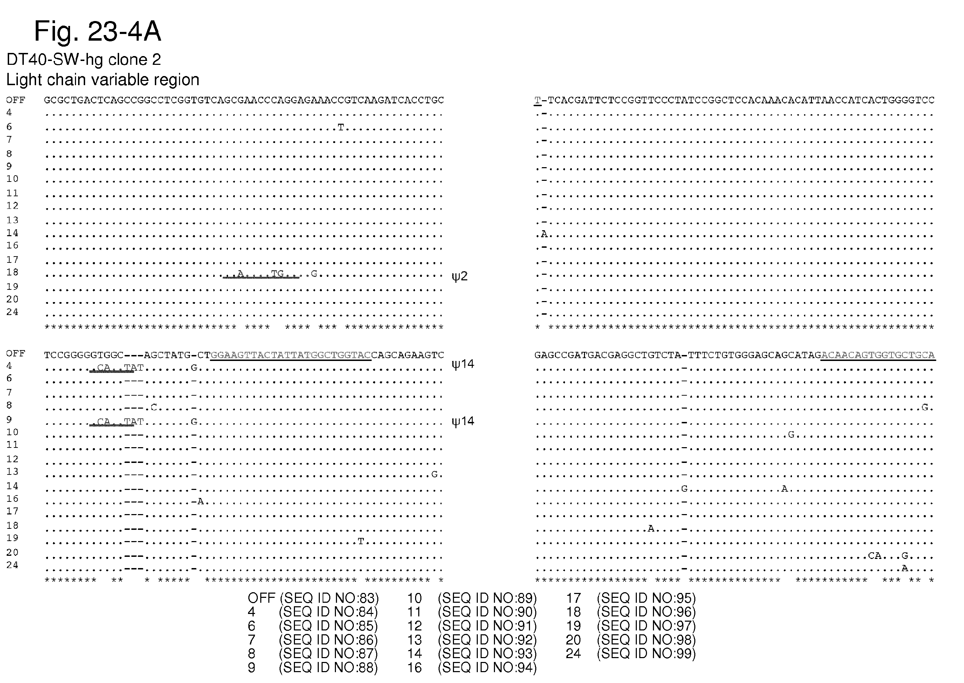

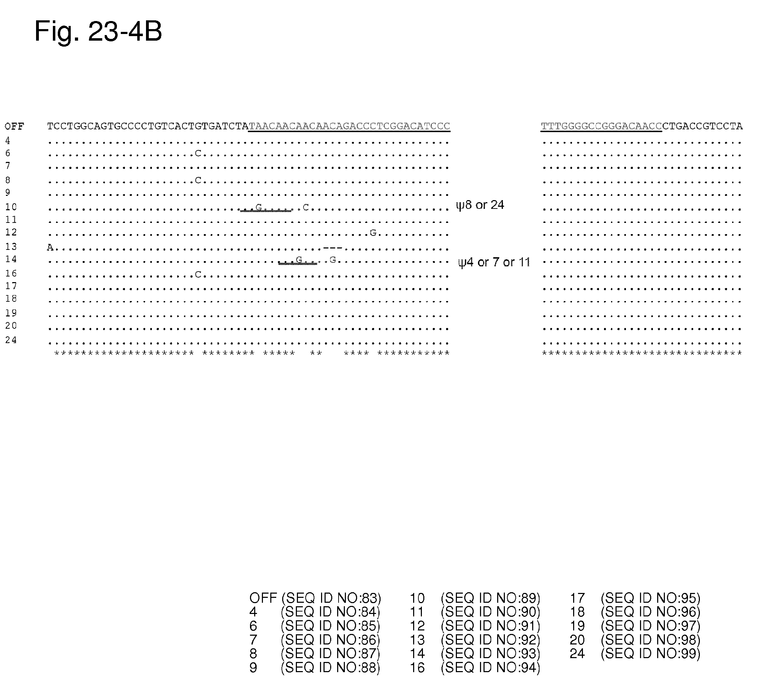

Fig. 23-4A shows that DT40-SW-hg introduced a mutation into an antibody variable region (light chain variable region) with high frequency (clone 2). Underlined parts indicate CDRs and genes described outside the columns are pseudo genes inferred to be used for gene conversion. The sequence of OFF, 4, 6, 7, 8, 9, 10, 11, 12, 13, 14, 16, 17, 18, 19, 20 and 24 are corresponding to SEQ ID NOs: 83 to 99, respectively.
Fig. 23-4B shows that DT40-SW-hg introduced a mutation into an antibody variable region (light chain variable region) with high frequency (continuation of Fig. 23-4A). The sequence of OFF, 4, 6, 7, 8, 9, 10, 11, 12, 13, 14, 16, 17, 18, 19, 20 and 24 are corresponding to SEQ ID NOs: 83 to 99, respectively.

### Embodiments for Carrying out the Invention

The B cells which produce a human-type antibody of the present invention are prepared using non-human-derived B cells as follows.

All cells may be used as non-human-derived B cells for preparation of the B cells which produce a human-type antibody in the present invention, as long as the cells express AID (activation-induced cytidine deaminase) (that is an enzyme playing an essential role in somatic hypermutation) in a constitutive or inductive manner, and have a property of introducing a spontaneous mutation into an antibody gene. Animal species and cell line types from which B cells are derived are not limited. B cells of a vertebrates such as a mouse, sheep, rat, rabbit, and chicken, or cell lines or mutant cell lines thereof can be used. Preferably, the chicken B cell-derived DT40 cell line is used. The cells of the DT40 cell line are cells of a B lymphoma-derived cell line, characterized by chromosome 2 trisomy (Baba, T.W., Giroir, B.P. and Humphries, E.H.: Virology 144: 139-151, 1985). As the DT40 cell line, a wild-type DT40 cell line or a DT40-SW cell line that the present inventors have established independently (a mutant cell line capable of reversibly switching the expression of the AID gene that governs mutation functions so as to be able to perform ON/OFF control of the mutation functions of an antibody (detailed information is described in Kanayama, N., Todo, K., Reth, M., Ohmori, H. Biochem. Biophys. Res. Common. 327: 70-75 (2005) and JP Patent Publication (Kokai) No. 2006-109711 A)) can be used.

These B cells are vertebrate B cells capable of inducing or halting AID (activation induced cytidine deaminase) expression with the induction of the expression of an exogenous Cre recombinase gene by extracellular stimulation followed by the inversion of the direction of the exogenous AID gene by expressed Cre recombinase, and the B cells are characterized in that: 1) the endogenous AID gene is functionally disrupted, and an AID protein resulting from the expression of the endogenous AID gene is not produced; 2) the B cells have an exogenous AID gene that is flanked by two loxP sequences in opposite directions to each other, and a promoter that is present upstream of the region flanked by the two loxP sequences and is capable of functioning in the animal cells,
when the AID gene is placed in a forward direction with respect to the promoter, the AID gene can be expressed by the promoter, and when the AID gene is placed in a reverse direction with respect to the promoter, the expression of the AID gene is halted; and 3) a Cre recombinase gene is introduced so that Cre recombinase activation is possible by extracellular stimulation, and the direction of the region flanked by the two loxP sequences, which contains the exogenous AID gene, is inverted by Cre recombinase activation.

To obtain the cells, first, cells are altered by functionally disrupting an endogenous AID gene of cells derived from an arbitrary vertebrate capable of expressing the endogenous AID gene, so as to prevent AID protein production as a result of the expression of the endogenous AID gene. One of the two alleles of the endogenous AID gene is disrupted (knockout) by constructing an AID gene targeting vector according to a general technique. For the other allele locus, an AID gene targeting vector containing a DNA construct that contains a gene encoding exogenous AID (constructed so that the direction can be inverted by Cre recombinase) is constructed. The endogenous AID gene is disrupted by causing homologous recombination. Thus, cells capable of controlling the expression of the exogenous AID gene can be prepared. The exogenous AID gene (constructed so that the direction can be inverted by Cre recombinase) is flanked by two loxP sequences having an inversion relationship. Furthermore, on the upstream side of the region flanked by the two loxP sequences, a promoter capable of functioning in subject animal cells is present. Hence, a targeting vector is preferably designed so that the promoter is also inserted onto the genome by homologous recombination. In this manner, such an exogenous AID gene (constructed so that the direction can be inverted by Cre recombinase) is incorporated into the genome of cells, so that when the exogenous AID gene is placed in a forward direction with respect to the above promoter, the promoter induces AID gene expression. Needless to say, when the AID gene is placed in a reverse direction with respect to the above promoter, AID gene expression does not take place. As promoters to be appropriately used herein, persons skilled in the art can conceive of and select various such promoters. Examples thereof include a β-action promoter, an immunoglobulin promoter, a cytomegalo virus promoter, and a CAG promoter.

For example, the above Cre recombinase gene is present in a form such that Cre recombinase causes the expression of a fusion protein with an estrogen receptor. The above extracellular stimulation is stimulation by estrogen or a derivative thereof. Hence, cells are stimulated extracellularly by estrogen or a derivative thereof, so that Cre recombinase activation is induced intracellularly. When cells are stimulated with an estrogen derivative, Cre recombinase activation is induced. Therefore, only when an estrogen derivative is caused to act extracellularly, Cre recombinase is activated (referring to a situation in which the above fusion protein is imported into nuclei, and thus Cre recombinase can act on loxP sequences) and the region containing the AID gene, which is flanked by loxP sequences, is inverted.

Furthermore, the region flanked by the above two loxP sequences may further contain a marker gene in the same direction as that of the AID gene. Here, when the AID gene is placed in a forward direction with respect to the promoter, the marker gene is expressed (since it is also placed in a forward direction), the AID gene is placed in a forward direction with respect to the promoter, and thus cells capable of expressing the AID gene can be selected by the marker.

Furthermore, the region flanked by the above two loxP sequences may further contain a marker gene in a reverse direction with respect to the AID gene. Here, when the AID gene is placed in a reverse direction with respect to the promoter, the marker gene is expressed since it is placed in a forward direction, the AID gene is placed in a reverse direction with respect to the promoter, and thus cells incapable of expressing the AID gene can be selected by the marker.

Such B cells can be obtained according to JP Patent No. 4487068.

For preparation of the B cells which producesproducing a human-type antibody of the present invention, the above antibody gene of non-human-derived B cells is substituted with a human antibody gene. At this time, an antibody constant region gene of non-human-derived B cells is substituted with a human antibody constant region gene.

Substitution for an antibody gene can be performed by homologous recombination. For substitution, an antibody gene of animal species is isolated from non-human B cells (to be used for preparation of the human-type antibody-producing cells of the present invention), the structure thereof is analyzed, and then a targeting vector for substitution of a constant region exon of the antibody gene is constructed. Subsequently, the above antibody gene of non-human B cells is substituted with a human antibody gene using the thus constructed targeting vector.

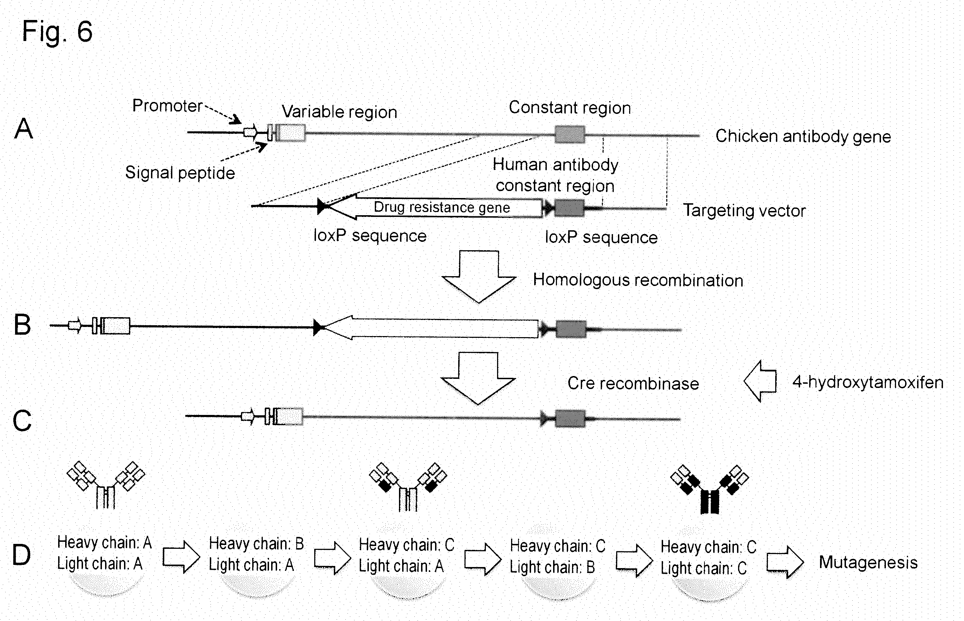

The outline of a specific method for introducing a human antibody constant region gene is as follows (Fig. 6). In the method described below, chicken DT40-SW is used.

### (1) Targeting vector

An about 2-kbp region upstream of and a 2-to-5 kbp region downstream of a constant region gene are used as regions required for homologous recombination.

As a splicing receptor sequence and a splicing branch point sequence contained in an intron upstream of a constant region gene, those of a human antibody gene are used.

As a polyA addition sequence downstream of a constant region gene, a chicken-derived polyA addition sequence is used.

A drug resistance gene flanked by loxP sequences is placed upstream of a human constant region gene containing a splicing sequence.

### (2) Preparation of chimeric antibody-producing cells

When the targeting vector is properly introduced into an antibody constant region gene, the structure of a wild-type (Fig. 6A) antibody gene is changed as shown in Fig. 6B. The antibody gene is divided by a drug resistance gene, so that the antibody gene is inactivated and thus is unable to result in antibody production. Therefore, the loss of antibody production on cell surfaces is analyzed by flow cytometry, in addition to drug resistance, the number of candidate cells, in which targeting has taken place as intended, can be narrowed down. Whether or not incorporation has taken place as expected can be confirmed by analysis of a genome gene using PCR or Southern blotting.

DT40-SW cells to be used for the method are caused to express Cre recombinase (Mer-Cre-Mer) as a fusion protein with a mutant estrogen receptor. Mer-Cre-Mer is generally in an inactive form, but can be activated by adding 4-hydroxytamoxifen (4-OHT) that is one of estrogen derivatives to a medium (Zhang, Y. et al., Nucleic Acids Res. 24 543-548 (1996)). Therefore, when 4-OHT is caused to act on cells (Fig. 6B) that have lost their antibody-producing capacity after introduction of a targeting vector, Cre recombinase is activated, a drug resistance gene flanked by loxP sequences is eliminated, and thus the structure is changed to an antibody gene structure as shown in Fig. 6C. In this structure, the expression of an antibody gene becomes possible again, and thus the expression of a chimeric antibody of the introduced chicken antibody variable region and human antibody constant region can be expected. Cells that have become possible to express such a chimeric antibody on cell surfaces can be isolated by flow cytometry or the like.

An antibody is composed of a heavy chain and a light chain. As shown in Fig 6D, gene recombination is performed gradually in order of light chain → heavy chain or heavy chain→light chain, and thus chimeric antibody-producing cells are prepared.

### (3) Mutagenesis at foreign antibody variable region gene

With human IgG1κ constant region-producing DT40-SW, mutagenesis at an antibody variable region gene is possible using procedures similar to those involving an *in vitro* method for producing an antibody using DT40-SW that has been established to date. In the cells, a mutagenesis mechanism is OFF at the initial state, but cells with their mutagenesis mechanism that have been turned ON by treatment with 4-OHT can be prepared. Cells with a mutagenesis mechanism turned ON express a green fluorescent protein, and thus separation by sorting is possible using flow cytometry. Subsequently, a cell population is generated only by continuing culture, in which a mutation has been introduced into a foreign antibody variable region. Cells producing an antibody having specificity and affinity of interest are isolated from the mutant library. Through repeated mutation and selection, affinity maturation of the obtained antibody is also possible. This method uses cells' own mutation capacity, so that consecutive mutation and selection can be performed easily. The properties of the thus obtained antibody can be exhibited by turning OFF the mutation functions of the cells via treatment with 4-OHT.

As described above, DT40-SW expressing a chimeric antibody with a human IgG1κ constant region can be prepared by the method without deteriorating the antibody mutation functions of DT40. The cells are referred to as "DT40-SE-hg." The cells can be used for antibody production in the same manner as that for conventional DT40-SW. The thus obtained human IgG1 antibody can be directly used for various types of evaluation concerning effector functions exhibited through mediation of a human antibody constant region. Thus, this is extremely useful as a technique for rapidly obtaining a candidate antibody for pharmaceutical development.

### Examples

The present invention is specifically described with reference to the following Examples, but is not limited to these Examples.

### (A) Substitution of chicken antibody light chain constant region gene with human antibody κ chain constant region gene

### 1) Construction of human κ chain constant region gene targeting vector

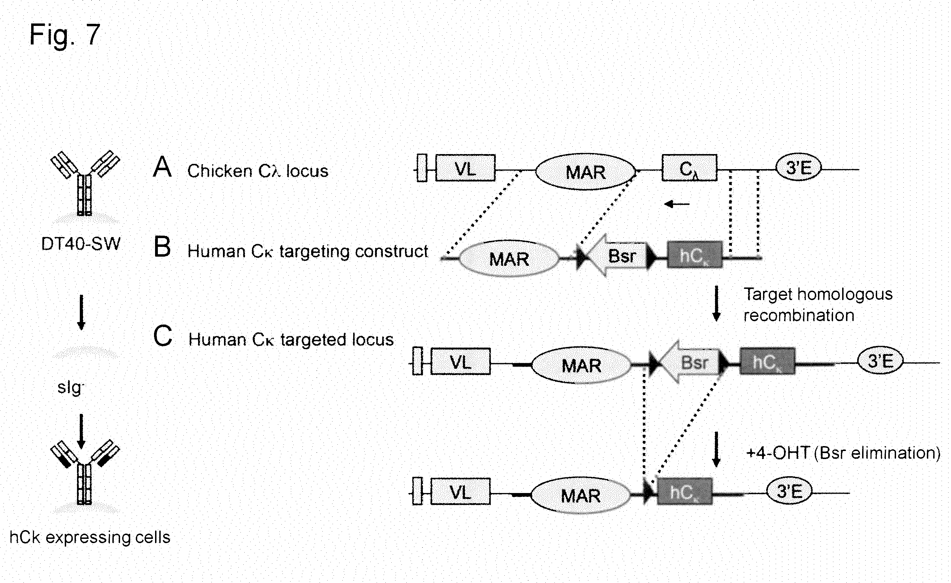

A method for substituting a chicken light chain constant region with a human κ chain constant region according to the outline shown in Fig. 6 is shown in Fig. 7. Upon construction of a targeting vector, the following points were particularly devised in addition to the above items: (i) a targeting arm was designed so as not to alter and/or delete a matrix attachment region (MAR) or a 3' enhancer (3' E), which are factors involved in gene expression and mutagenesis; (ii) a fragment containing the human κ chain constant region (Cκ) gene to be incorporated into the vector contained a splicing acceptor sequence and a branch point sequence in an intron in a upstream portion of human Cκ; (iii) a human Cκ gene fragment to be incorporated into the vector contained no polyA addition sequence so that a polyA sequence on the chicken antibody light chain gene was used.

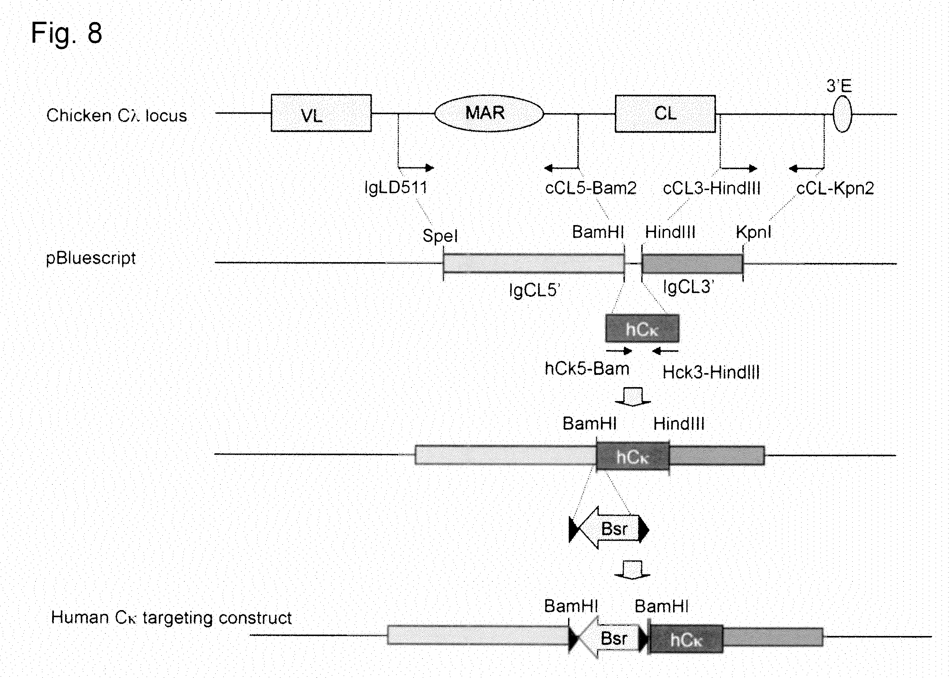

Procedures for construction of a targeting vector are as described below (Fig. 8). The chicken light chain gene has already been cloned (Reynaud, C.-A., et al., Cell 40, 283-291 (1985)), and the surrounding nucleotide sequences have also been revealed by genome analysis (International Chicken Genome Sequencing Consortium, Nature 432, 695-716 (2004)). To obtain a gene fragment surrounding the chicken antibody light chain variable region, primers were designed based on sequences that had been revealed, PCR was performed to obtain a gene fragment. From the genomic DNA of DT40-SW, a gene fragment to be used for the 5' targeting arm (IgCL5') upstream of the constant region was amplified by PCR using primers, IgLD511 (5'-GAGTCGCTGAACTAGTCTCGGTCTTTCTTCCCCCATCG-3' (SEQ ID NO: 5), ACTAGT (*Spe* I site)) and cCL5-Bam2 (5'-ACGGATCCATATCTATTTTCATGGATGTTATACGTGTGCG-3' (SEQ ID NO: 6), GGATCC *(BamH* I site)), and KOD-Plus DNA polymerase (Toyobo Co., Ltd.). After treatment with *Spe* I and *BamH* I, the resultant was incorporated into BluescriptII SK (-). Similarly, a gene fragment to be used for the 3' targeting arm (IgCL3') downstream of the constant region was amplified using primers, cCL3-*Hin*d III (5'-CTAAGCTTCCCACTGGGGATGCAATGTGAGGACAGT-3' (SEQ ID NO: 7), AAGCTT (*Hin*d III site)) and cCL3-Kpn2 (5'-TGCGATCCAGGTACCACGATAGCACTGCCTGCCTCCATC-3' (SEQ ID NO: 8), GGTACC (*Kpn* I site)). After treatment with *Hind* III and *Kpn* I, the resultant was incorporated into a site downstream of IgCL5' in pBluescriptII SK(-). Furthermore, from the genomic DNA of human Burkitt's lymphoma cell line Daudi (obtained from Riken cell bank), a gene fragment containing hCk was amplified using primers hCk5-Bam (5'-CTAAACTCTGAGGGGATCCGATGACGTGGCCATTCTTTGC-3' (SEQ ID NO: 9), GGATCC *(BamH* I site)) and hCk*-Hin*d III (5'-GTAAGCTTCTAACACTCTCCCCTGTTGAAGCTCTTTGTGA-3' (SEQ ID NO: 10), AAGCTT (*Hin*d III site)). After treatment with *Bam*H I and *Hin*d III, the resultant was inserted into a site between IgCL5' and IgCL3'. Furthermore, Blasticidin S resistance gene (Bsr) having loxP sequences on both ends (Arakawa, H., et al. BMC Biotechnol. 1, 7 (2001)) was incorporated into a *Bam*H I site immediately upstream of hCk.

### 2) Preparation of DT40-SW with human κ chain constant region gene targeting vector incorporated therein

The human Cκ gene targeting vector (15 µg) constructed in (A)-1) was cleaved with *Not* I for linearization. The resultant was mixed with 1×10⁷ DT40-SW cells to prepare 500 µl of a suspension. The suspension was added to a 4-mm gap electroporation cuvette, and then electroporation was performed under conditions of 550 V and 25 µF. A Gene Pulser Xcell (Bio-Rad Laboratories Inc.) was used for electroporation. After electroporation, cells were suspended and then cultured for 24 hours in 10 ml of growth medium (PRMI1640, Invitrogen; 10% fetal calf serum, Invitrogen; 1% chicken serum, Sigma). Ten (10) ml of 2 × selective medium (growth medium + blasticidin S (Kaken Pharmaceutical Co., Ltd.)) was added to prepare blasticidin S having a final concentration of 20 µg/ml, and then the solution was dispensed to two 96-well plates, followed by 10 to 14 days of culture. The procedure was attempted 4 times, so that colonies were selected with the use of blasticidinS and thus 139 clones were obtained.

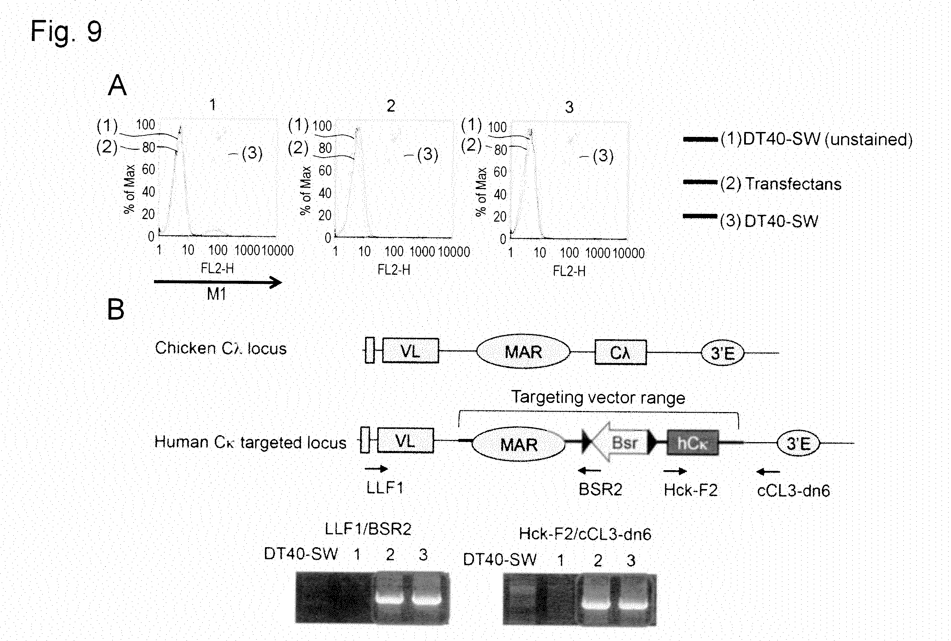

Cells that had formed colonies were stained with R-phycoerythrin (R-PE) labeled mouse anti-chicken IgM mAb (clone M1, Southern Biotechnology), and then analyzed using a FACS Calibur (BD Bioscience). As intended, targeted cells could be expected to be able to express an antibody heavy chain. Hence, 3 clones not stained with an anti-chicken IgM antibody were selected (Fig. 9A). For confirmation at the genetic level, genomic DNA was extracted from these cells, and incorporation of a targeting vector was confirmed by PCR using a primer for the outer region of the targeting vector and a primer specific to the vector. Specifically, incorporation of a targeting vector was confirmed by PCR using, in the case of an upstream region, primers LLF1 (5'-CCATCGGCGTGGGGACACACAGCTGCTGGGATTCC-3' (SEQ ID NO: 11)) and BSR2 (5'-GTGATGATGAGGCTACTGCTGACTCTCAACATTCTACTCCTCC-3' (SEQ ID NO: 12)), and in the case of a downstream region, Hck-F2 (5'-GAGAGGCCAAAGTACAGTGGAAGGTGGATAACGCCCTCC-3' (SEQ ID NO: 13)) and cCL3-dn6 (5'-GGAGCTGTACCATGCGGCCTGCTCTGCTGATGCCATGTCG -3' (SEQ ID NO: 14)) (Fig. 9B). Specific amplification of PCR products was observed in clones 2 and 3, indicating that targeting had been achieved as intended. Of these clones, clone 2 was used for the following procedures.

### 3) Expression of human κ chain constant region

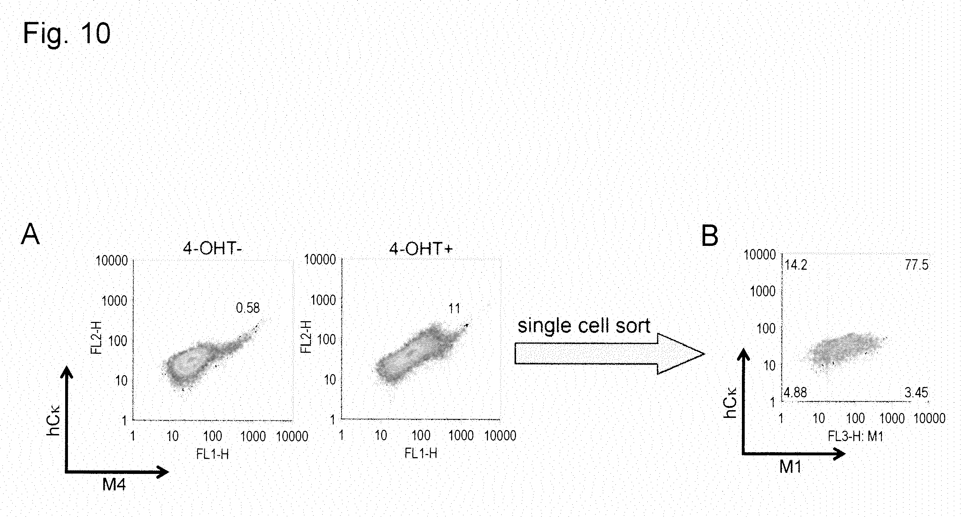

As reported previously for clone 2, drug resistance genes were eliminated by treatment with 50 nM 4-OHT (Kanayama, N., et al. Biochem. Biophys. Res. Commun. 327, 70-75 (2005)). Cells from which drug resistance genes had been eliminated were expected to recover antibody expression therein. Antibody expression on cell surfaces was analyzed by flow cytometry after staining with a mouse anti-chicken IgM mAb clone M1 or clone M4 (Riken Cell Bank) and mouse anti-human Igk light chain (BD Bioscience). As a result, the appearance of cells expressing both IgM and human κ chain on cell surfaces was confirmed (Fig. 10A). Cells expressing human Cκ at high levels were subjected to single cell sorting using FACS Aria, revealing that these cells expressed chicken IgM and human Cκ on cell surfaces at high expression levels. Moreover, secretion of an antibody resulting from association of chicken µ chain with human κ chain in the culture solution was confirmed by ELISA. All the confirmed clones had lost their drug resistance. Of these clones, a typical clone was designated as DT40-SW-hk and then examined as follows.

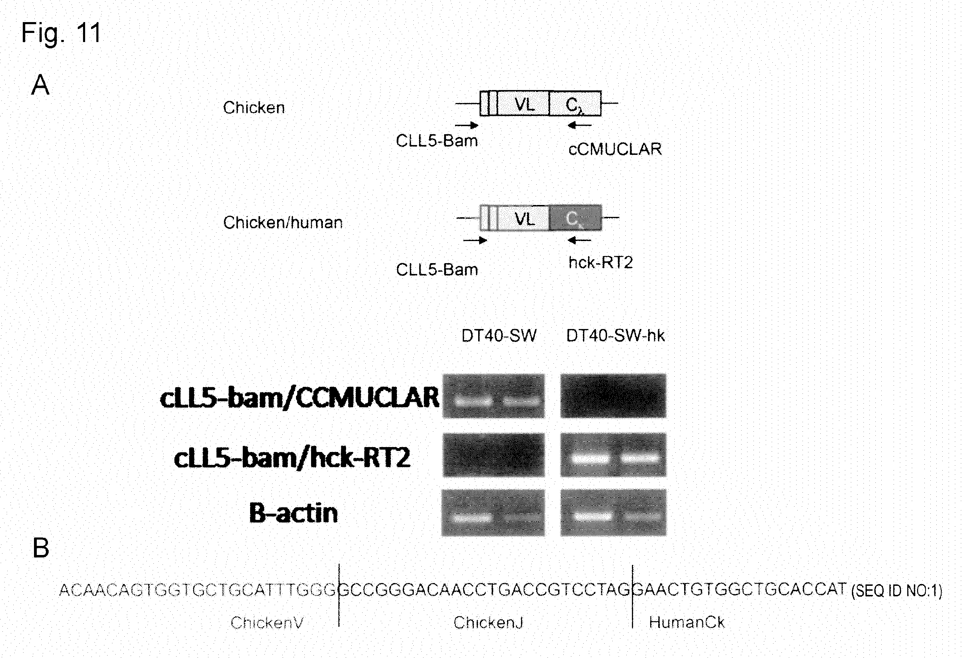

The expression of human Cκ gene (incorporated into DT40-SW-hk) at the transcriptional level was analyzed by RT-PCR. Total RNA of DT40-SW-hk was extracted using TRIzol (Invitrogen), and then cDNA was synthesized using Superscript II reverse transcriptase (Invitrogen) and an oligo dT primer. With the use of the cDNA as a template, a sense primer (CLL5-Bam: 5'-CGGCGTGGGGATCCACAGCTGCTGGGATTC-3' (SEQ ID NO: 15)) and an antisense primer (cCMUCLAR: 5'-GGAGCCATCGATCACCCAATCCAC-3' (SEQ ID NO: 16)) or (hck-RT2: 5'-CTCATCAGATGGCGGGAAGATGAAGACAGATGGTGCAGCC-3' (SEQ ID NO: 17)) were used for amplification of a light chain. Moreover, as an internal standard, a β actin transcription product was amplified using primers actin3 (5'-CTGACTGACCGCGTTACTCCCACAGCCAGC-3' (SEQ ID NO: 18)) and actin4 (5'-TTCATGAGGTAGTCCGTCAGGTCACGGCCA-3' (SEQ ID NO: 19)). As a result, it was confirmed that DT40-SW-hk did not express the chicken antibody light chain, but expressed a chimeric transcription product of the chicken light chain variable region and the human κ chain constant region (Fig. 11A). Furthermore, when the amplification products were subjected to sequence analysis, it was confirmed that exons had been bound normally, and the resultant was the transcription product encoding the antibody light chain protein as intended (Fig. 11B).

### (B) Substitution of chicken antibody µ chain gene with human antibody γ 1 chain gene

### 1) Construction of human γ 1 chain gene targeting vector

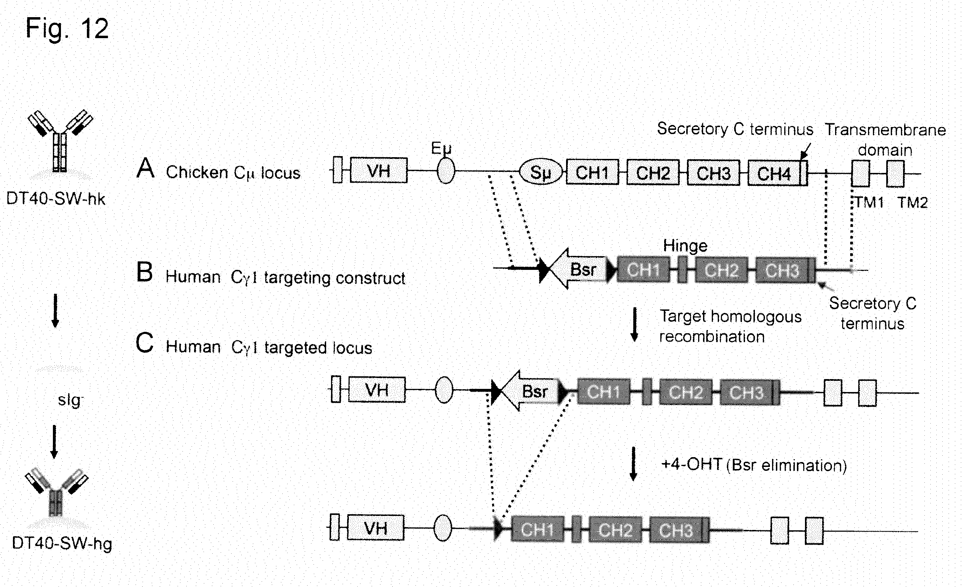

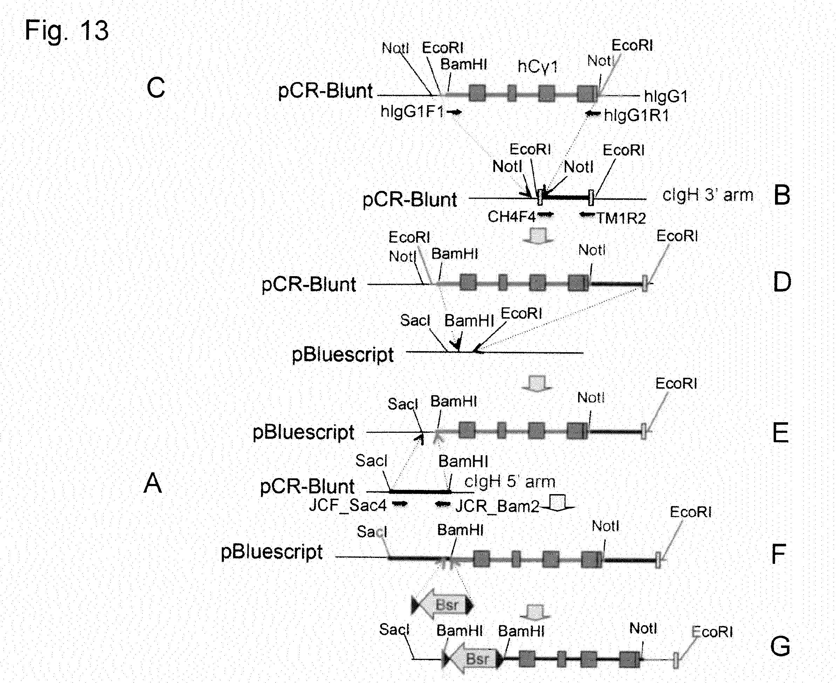

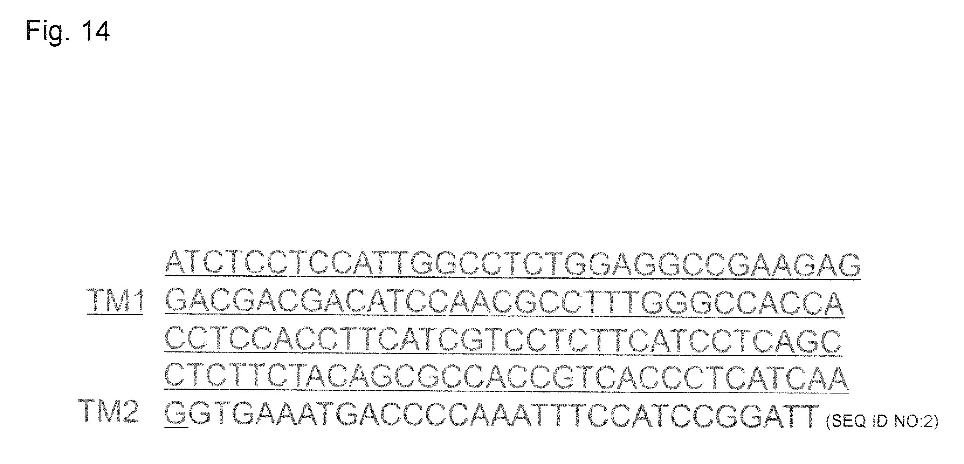

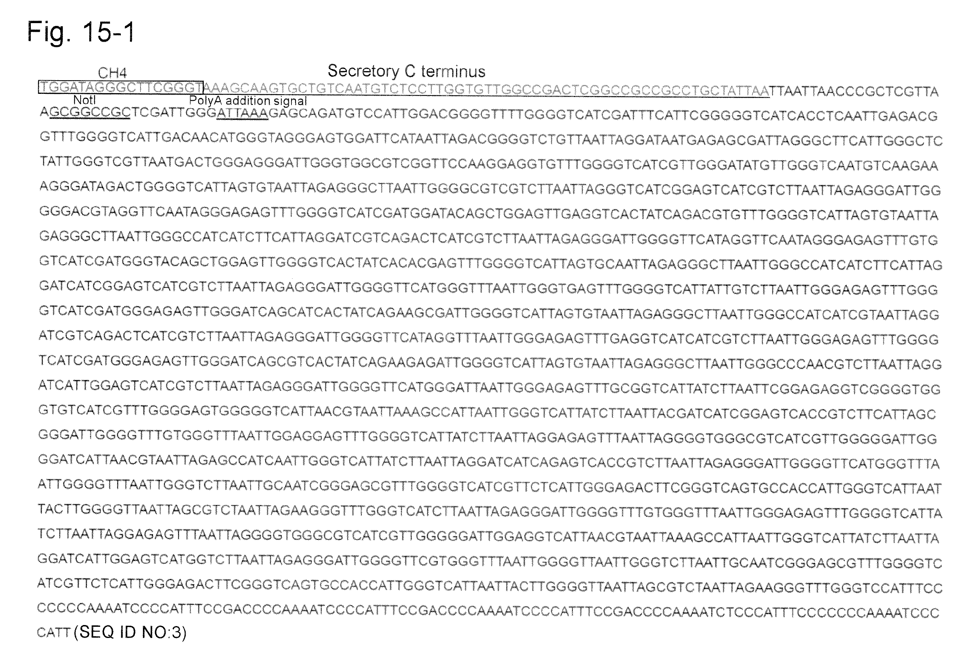

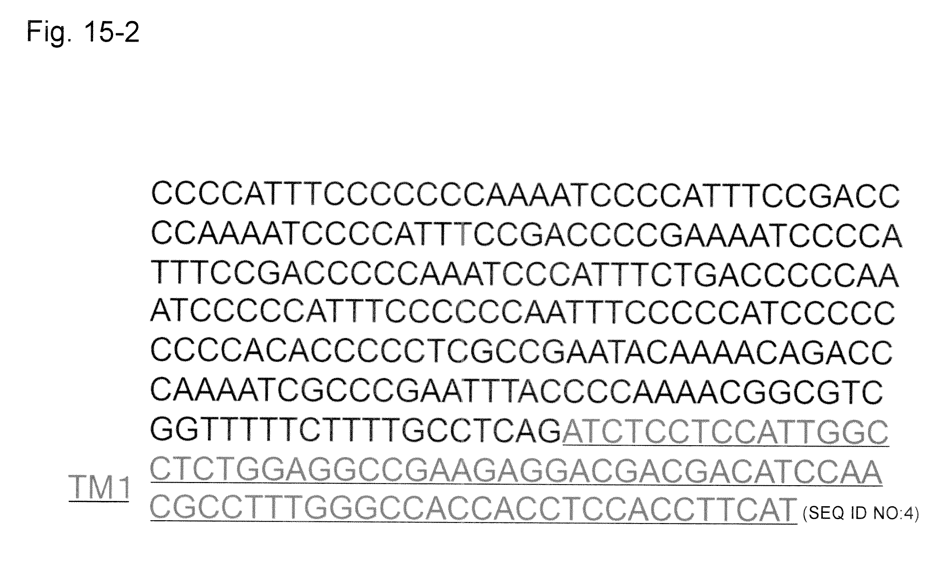

According to the outline shown in Fig. 4, a method for substitution of the chicken µ chain (Cµ) gene with the human γ 1 chain (Cγ1) gene is as shown in Fig. 12. Upon construction of a targeting vector, the following points were devised: (i) a targeting arm was designed so as not to alter and/or delete a µ enhancer (Eµ) that is a factor involved in gene expression and mutagenesis; (ii) the 5' targeting arm was designed, so that it was placed in a region upstream of Sµ since a switch region (Sµ) (containing many repetitive sequences) is present immediately upstream of the constant region; (ii) CH1, CH2, CH3, CH4, and secretory C terminal exon of the chicken Cµ gene were substituted with CH1, hinge, CH2, CH3, and secretory C terminal exon of the human Cγ1 gene, and a transmembrane domain exon of the chicken µ chain gene was left for use; (iii) the human Cγ1 gene fragment contained a splicing acceptor sequence and a branch point sequence in the intron in the Cγ1 upstream portion; and (iv) the human Cγ1 gene fragment contained no polyA addition sequence downstream of the secretory C terminus, so that a polyA sequence downstream of the secretory C terminus of chicken Cµ was used. Procedures for construction of the targeting vector are as described below (Fig. 13). To obtain a gene fragment to be used for the 5' targeting arm (cIgH 5' arm) in a region upstream of Sµ, primers JCFF_Sac4 (5"- AAATGGCCGAATTGAGCTCGGCCGTTTTACGGTTGGGTTC -3' (SEQ ID NO: 20), GAGCTC was *Sac* I site) and JCR_Bam2 (5'-CTCATCATTCAGTATCGATGGATCCTTAATTACTCCCACG -3' (SEQ ID NO: 21), GGATCC was *Bam*H I site) were designed from nucleotide sequences in the periphery of Sµ (Kitao, H. et al. Int. Immunol. 12, 959 (2000); accession no. AB029075), followed by amplification by PCR from the genomic DNA of DT40-SW. The thus obtained gene fragment was incorporated into pCR-Blunt (Invitroten), and then the nucleotide sequence was confirmed (Fig. 13A). Information concerning genes downstream of the secretory C terminal exon of the chicken Cµ gene to be used for the 3' targeting arm (cIgH3' arm) has not yet been published. Hence, from the mRNA sequence of chicken secretory IgM (Dahan, A. et al. Nuclec Acids Res. 11, 5381 (1983); accession no. X01613), a sense primer cCmu4-F1 for the CH4 domain (5'-GGCTCAGCGTCACCTGCATGGCTCAGG-3' (SEQ ID NO: 22)) was designed. The gene fragment was amplified by PCR using the sense primer with a primer mCmyu3' (5'-CCTTGATTTCGAAGTGGAGAAGACGTCGGGAGGTGGAGA-3' (SEQ ID NO: 23); Sayegh, C. E., et al. Proc. Natl. Acad. Sci. U.S.A. 96, 10806 (1999)) for 3' UTR downstream of a transmembrane region gene of membrane-type IgM. The resultant was cloned to pCR-Blunt, so as to reveal the sequences of transmembrane region genes encoded by TM1 exon and TM2 exon and a partial sequence of 3' UTR (Fig. 14). On the basis of the nucleotide sequence of chicken secretory IgM and TM1 sequence shown in Fig. 14, primers CH4F4 (5'-TGGATAGGGCTTCGGGTAAAGCAAGTGCTGTCAATGTCTC-3' (SEQ ID NO: 24)) and TM1R2 (5'-ATGAAGGTGGAGGTGGTGGCCCAAAGGCGTTGGATGTCG-3' (SEQ ID NO: 25)) were designed. Amplification was performed by PCR using these primers and the genomic DNA between CH4 and TM1 of the chicken Cµ gene. For amplification of the fragment, KOD-FX (Toyobo Co., Ltd.) was used. The thus obtained gene fragment was cloned into pCR-Blunt, and then partial sequences were determined from the 5' side and the 3' side (Fig. 13B and Fig. 15). The human Cγ1 gene fragment was amplified using the genomic DNA of the human Burkitt's lymphoma cell line Daudi as a template and primers hIgG1F1 (5'-ACGGATCCTGCAAGCTTTCTGGGGCAGGCCAGGCCTGACC-3' (SEQ ID NO: 26), GGATCC (*Bam*H I site)) and hIgG1R1 (5'-CGGCGGCCGCACTCATTTACCCGGAGACAGGGAGAGGCTC-3' (SEQ ID NO: 27), GCGGCCGC was *Not* I site). The resultant was cloned into pCR-Blunt and then the sequence was confirmed (Fig. 13C). A *Not* I fragment containing the hCγ1 gene was inserted to a *Not* I site upstream of the cIgH3' arm (Fig. 13D), and then a *Bam*H I-*Eco*R I fragment prepared by ligating the cIgH3' arm to the hCγ1 gene was inserted to pBluescriptII SK(-) (Fig. 13E). Furthermore, the cIgH 5' arm was inserted into the *Sac* I-*Bam*H I region upstream of hCγ1 (Fig. 13F). Finally, a blasticidin resistance gene flanked by loxP sequences was inserted to *BamH* I between the cIgH5' arm and hCγ1 (Fig. 13G).

### 2) Preparation of DT40-SW with human γ1 chain gene targeting vector incorporated therein

The human Cκ1 gene targeting vector (15 µg to 40 µg) constructed in (B)-1) was cleaved with *Not* I for linearization. The resultant was mixed with 1×10⁷ DT40-SW cells to prepare 500 µl of a suspension. The suspension was added to a 4-mm gap electroporation cuvette, and then electroporation was performed under conditions of 550V or 700V, and 25 µF. Gene Pulser Xcell was used for electroporation. After electroporation, cells were suspended and then cultured for 24 hours in 10 ml of growth medium and then suspended in 20 ml of growth medium containing blasticidin S with a final concentration of 20 µg/ml. The solution was dispensed to two 96-well plates, followed by 10 to 14 days of culture. The procedure was attempted 17 times, so that colonies were selected with the use of blasticidinS and thus 207 clones were obtained.

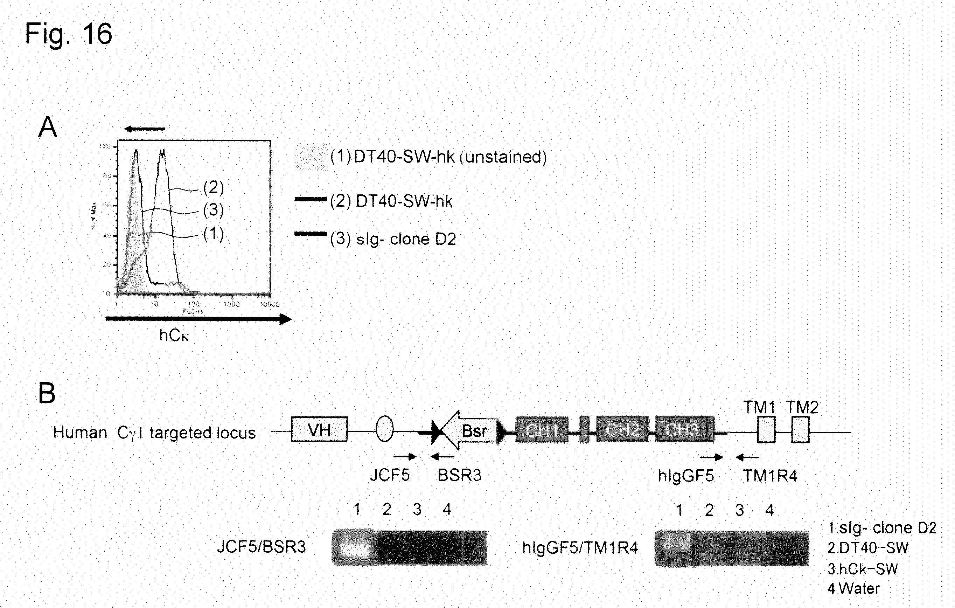

Cells that have been targeted as intended can be expected to become unable to express an antibody heavy chain. Hence, cells that had formed colonies were analyzed by FACS Calibur after staining with R-PE labeled mouse anti-chicken IgM mAb clone M1. As a result, 1 clone not stained with the anti-chicken IgM antibody was obtained (Fig. 16A).

For confirmation at the genetic level, genomic DNA was extracted from these cells. Incorporation of the targeting vector was confirmed by PCR using, a primer for the outer region of the targeting vector and a primer specific to the vector. Specifically, incorporation of the vector was confirmed by PCR using, in the case of an upstream region, primers JCF5 (5'-TAATCGGTACTTTTTAATCCTCCATTTTGCCCGAAATCGC-3' (SEQ ID NO: 28)) and BSR3 (5'-CTGTGGTGTGACATAATTGGACAAACTACCTACAGAG-3' (SEQ ID NO: 29)), and in the case of a downstream region, hIgGF5 (5'-TGGACTCCGACGGCTCCTTCTTCCTCTACAGC-3' (SEQ ID NO: 30)) and TM1R4 (5'-CCTTGATGAGGGTGACGGTGGCGCTGTAGAAGAGGCTG-3' (SEQ ID NO: 31)) (Fig. 16B). For clone D2 (that is, sIg-), specific amplification of PCR products indicating that targeting had been performed as intended was observed. In this attempt, the efficiency for obtaining cells of interest was extremely poor, but the subsequent optimization of transfection conditions resulted in establishment of an efficient method for establishing cell lines.

### 3) Expression of human IgG1 antibody

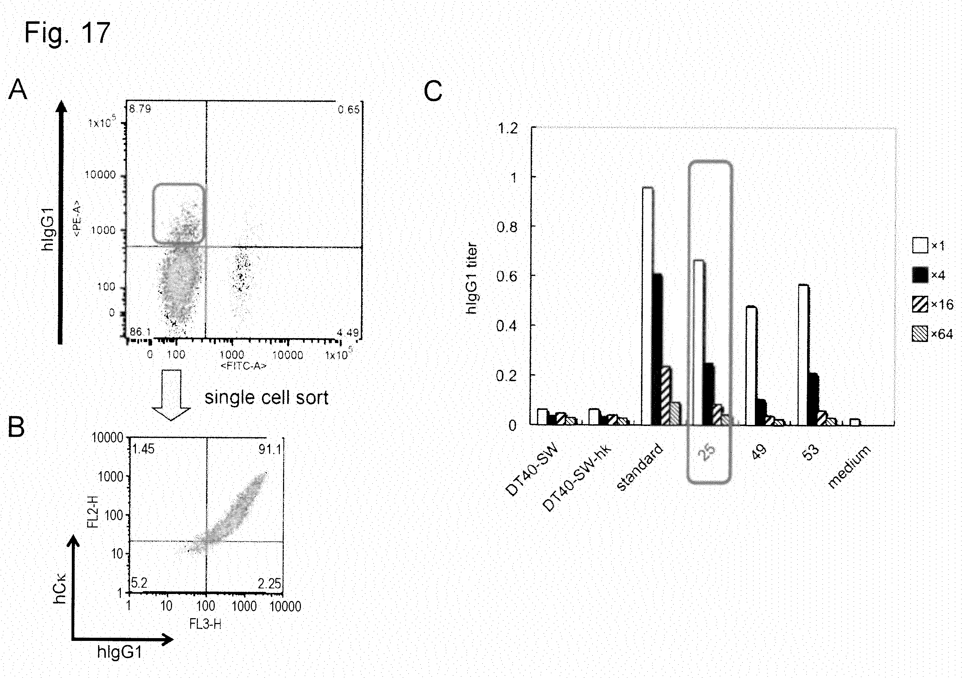

Clone D2 was treated with 50 nM 4-OHT, so as to eliminate drug resistance genes. Cells from which drug resistance genes had been eliminated were expected to recover antibody expression. Antibody expression on cell surfaces was analyzed by flow cytometry after staining with biotinylated mouse anti-human IgG1 mAb (ZYMED) and PE-Cy™5 Streptavidin (BD Pharmingen). The appearance of cells expressing human IgG1 on cell surfaces was confirmed (Fig. 17A). Cells expressing human IgG1 at high levels were subjected to single cell sorting using FACS Aria. The thus obtained colonies were subjected to flow cytometric analysis, so that 3 clones expressing human IgG1 and human κ chain at high levels were selected (clones 25, 49, 53) (Fig. 17B). The fact that these clones had lost drug resistance genes was confirmed by PCR. Antibody secretion by these clones in culture supernatants was examined by ELISA. Goat anti-human IgG Fc (Betyl) diluted 500-fold was bound onto a 96-well plate (Greiner), serial dilution was performed, human IgG1 antibody (in a sample supernatant) bound onto the plate was detected using peroxidase-conjugated goat anti-human IgG Fc (KPL) diluted 500-fold. As a result, 3 clones were confirmed to exhibit secretion levels equivalent to each other. It was demonstrated that human IgG1 antibody-producing DT40 prepared by the method can express both membrane-type and secretory antibodies (Fig. 17C). Of these clones, clone 25, the typical one, was designated as DT40-SW-hg and then subjected to detailed examination.

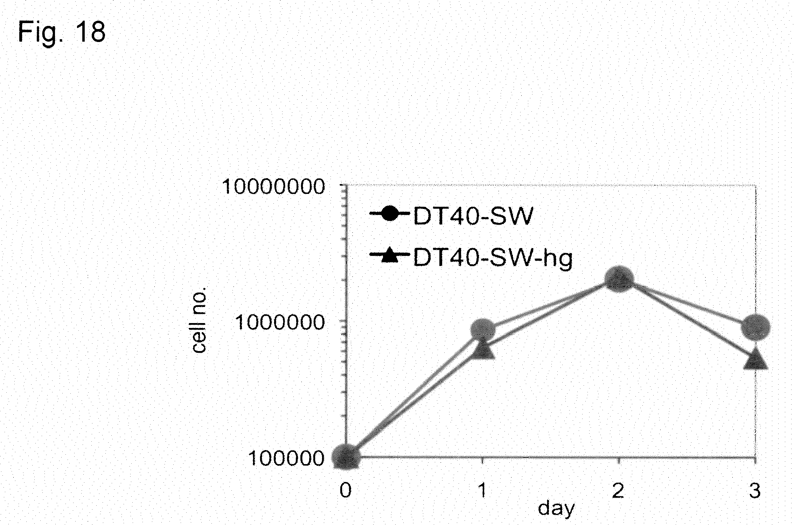

The proliferation capacity of DT40-SW-hg was compared with that of DT40-SW. DT40-SW-hg exhibited proliferation equivalent to that of DT40-SW (Fig. 18). Specifically, it was confirmed that the expression of a human-type antibody did not affect proliferation processes important for mutagenesis, even when mutation is turned ON and cells were cultured for a long time period.

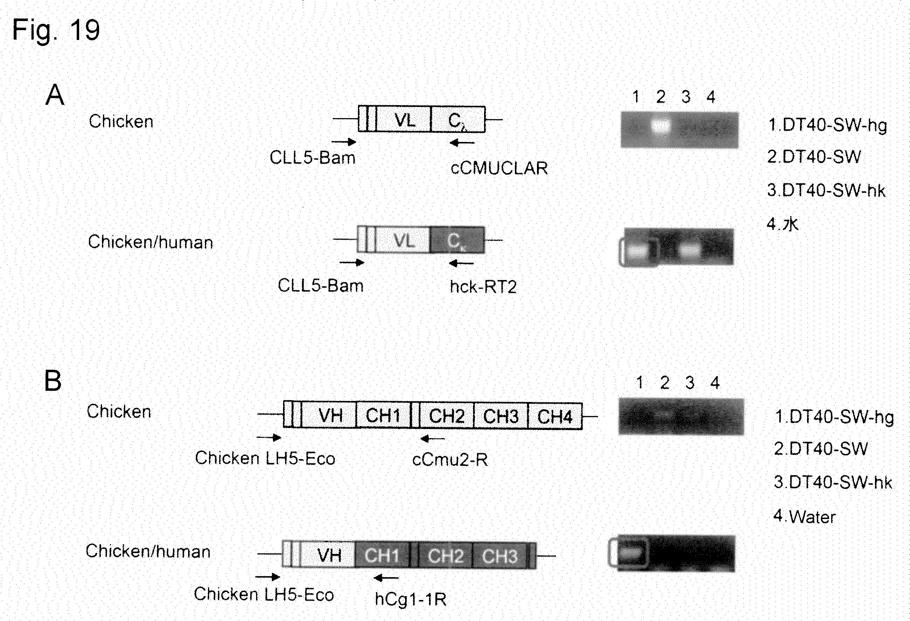

The expression of the human Cγ1 gene and the human Cκ gene incorporated into DT40-SW-hg was analyzed by RT-PCR at the transcriptional level. Total RNA of DT40-SW-hg was extracted using TRIzol, cDNA was synthesized using Superscript II reverse transcriptase and oligo dT primer. The light chain was amplified using the cDNA as a template, a sense primer CLL5-Bam, and an antisense primer cCMUCLAR or hck-RT2. The heavy chain was amplified using a sense primer Chicken LH5-Eco (5'-GCCGGAATTCCGACGGAGGAGCACCAGTCG-3' (SEQ ID NO: 32)) and an antisense primer cCmu2-R (5'-TTGTACCACGTGACCTCGGTGGGACGGCG-3' (SEQ ID NO: 33)) or hCg1-1R (5'-CTCTTGGAGGAGGGTGCCAGGGGGAAGACC-3' (SEQ ID NO: 34)). As a result, it was confirmed that DT40-SW-hg did not express the chicken antibody, but expressed a chimeric transcription product of the chicken antibody variable region and the human antibody constant region (Fig. 19).

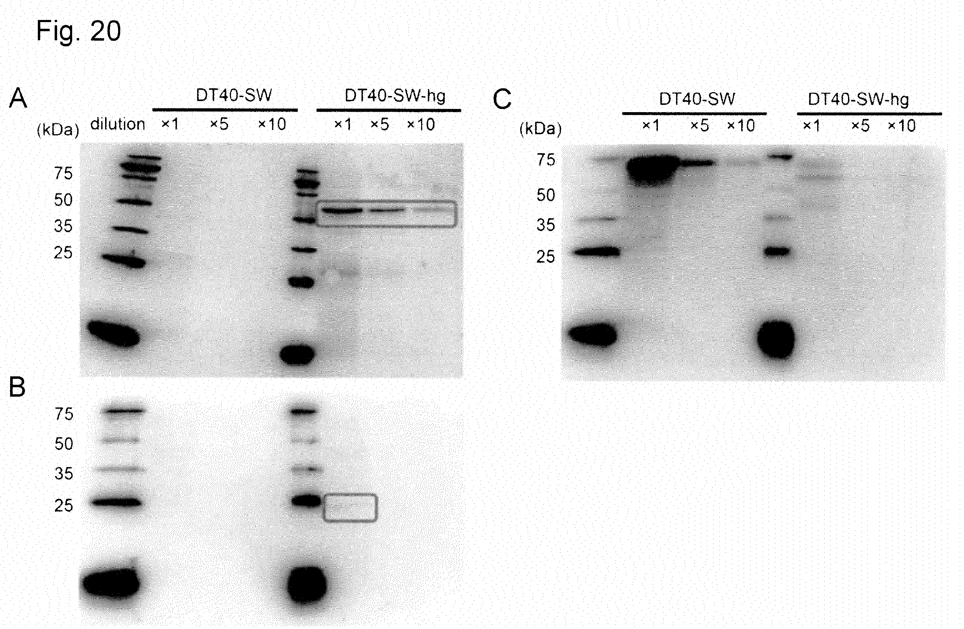

Western blotting was performed to confirm the expression of the human Cγ1 gene and the human Cκ gene incorporated into DT40-SW-hg on cell surfaces and within cells at the protein level. The thus collected cells were washed with PBS(-), and then suspended at 5×10⁶ cells/200 µl in PBS(-) supplemented with 0.1% TritonX100 and 1/100 volume of protease inhibitor (nacalai tesque). After 15 seconds of vortexing, the solution was left to stand for 30 minutes on ice. During the procedure, the solution was stirred every 10 minutes. After 10 minutes of centrifugation at 12,000 rpm, the supernatant was collected so as to prepare a cell-free extract. The protein concentration in the cell-free extract was determined using a BCA Protein Assay Kit (PIERCE), and then 50 µg or 150 µg of the cell-free extract was separated by 12.5% SDS-PAGE. The thus separated protein was transferred to a polyvinylidene fluoride (PVDF) membrane (Immobilon-P, Millipore) for reaction with each antibody, and then detected using an ECL Advance Western Blotting Detection Kit. For chemiluminescence, data were retrieved using a ChemiDoc XRS system (Bio-Rad Laboratory). Regarding antibodies, for detection of human IgG Fc, peroxidase labeled goat anti-human IgG Fc diluted 5000-fold was used. For detection of human κ chain, biotinylated mouse anti-human Ig k light chain diluted 200-fold (BD pharmigen) and streptavidin-HRP Conjugate diluted 100000-fold (ZYMED) were used. For detection of chicken IgM, peroxidase conjugated goat anti-chicken IgM diluted 10000-fold (mu chain) (ROCKLAND) was used. As a result, it was demonstrated that DT40-SW-hg expressed a heavy chain containing about 55-kDa human IgG1 Fc and a light chain containing about 25-kDa human Cκ, but did not express chicken IgM (Fig. 20). The molecular weight of the heavy chain of the chicken-human chimeric antibody presumed from the amino acid sequence was 56 kDa and the same of the light chain was 28 kDa. Thus, the expression of the protein having a molecular weight almost as predicted was confirmed.

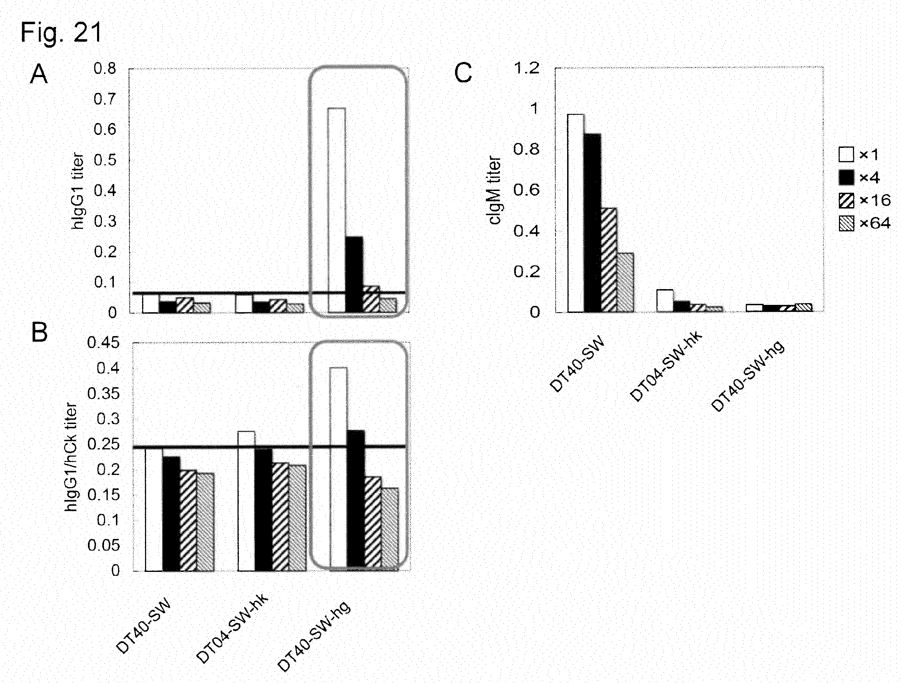

Furthermore, not only the secretion of the human IgG1 antibody from DT40-SW-hg was confirmed by ELISA using an antibody against a Fc part, but also the association of the human γ1 chain with the human κ chain was examined by ELISA using each specific antibody (Fig. 21). Association of the human γ1 chain with the human κ chain was detected using goat anti-human IgG Fc (500-fold dilution), biotinylated mouse anti-human Igk light chain (500-fold dilution), and streptavidin-HRP conjugate (1000-fold dilution). Chicken IgM was detected using goat anti-chicken IgM (mu chain) (Betyl) (500-fold dilution) and peroxidase-conjugated goat anti-chicken IgM (mu chain) (ROCKLAND) (1,000-fold dilution). The secreted antibody was analyzed using a culture supernatant subjected to serial dilution. DT40-SW-hg did not secrete chicken IgM (Fig. 21C), but secreted only the human IgG1 antibody (Fig. 21A). The secreted antibody was confirmed to be in a form such that the human γ1 chain was associated with the human κ chain (Fig. 21C). Specifically, it was demonstrated that DT40-SW-hg is a useful cell line capable of expressing a chimeric antibody, wherein the chimeric heavy chain and the chimeric light chain of the chicken antibody variable region and the human antibody constant region are stably associated with each other, on cell surfaces and in culture supernatants.

### (C) Mutagenesis at antibody variable region in human IgG1 antibody-producing DT40-SW (DT40-SW-hg)

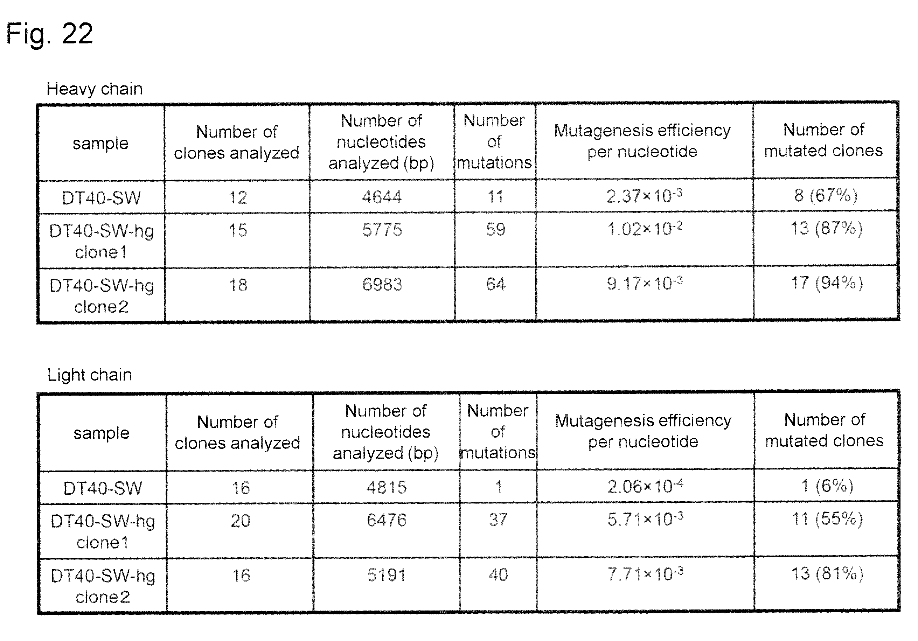

The mutation mechanism of DT40-SW-hg was turned ON by the previously reported method and then mutagenesis at an antibody variable region was analyzed (Kanayama, N., et al. Biochem. Biophys. Res. Commun. 327, 70-75 (2005)). After treatment with 4-OHT, GFP⁺ cells; that is, cells with AID expression (essential for mutagenesis) turned ON were subjected to single cell isolation by flow cytometry using FACSAria. The two independent clones were cultured for 41 days while being maintained under conditions of a scale of 5 ml, 5×10⁵ cells or more, and no over growth. Genomic DNA was isolated from the cultured cells. The heavy chain variable region was amplified by PCR using primers CVH1F2 (5'-GGCGGCTCCGTCAGCGCTCTCT-3' (SEQ ID NO: 35)) and CJH1R2 (5'-GCCGCAAATGATGGACCGAC-3' (SEQ ID NO: 36)). The light chain variable region was amplified by PCR using primers CVLF61 (5'-GGCACGGAGCTCTGTCCCATTGCTG-3' (SEQ ID NO: 37)) and CVLR31 (5'-CCCCAGCCTGCCGCCAAGTCCAAG-3' (SEQ ID NO: 38)). Each resultant was cloned into a pCR-Blunt vector. Plasmid DNA was extracted using a High Pure Plasmid Isolation Kit (Roche). Sequencing reaction was conducted using an AB1 PRISM Big Dye Terminator Cycle Sequencing Ready Reaction Kit (Applied Biosystems). Then the nucleotide sequence was analyzed using an ABI PRISM 310 Genetic Analyzer (Applied Biosystems). The nucleotide sequence of a sample after 41 days of culture was compared with the original nucleotide sequence before turning mutation ON. As a a result, many mutations were introduced in both the heavy chain and light chain of the antibody variable region gene of DT40-SW-hg, compared with the original DT40-SW cell line (Fig. 22). In DT40-SW-hg, high-frequency mutagenesis was confirmed compared with DT40-SW, in terms of both mutagenesis efficiency per nucleotide and the proportion of clones having mutations among all clones analyzed. It was suggested that DT40-SW-hg has sufficient mutation capacity for construction of an antibody library. Furthermore, as shown in the distribution of mutations introduced in the variable region gene, mutations were introduced into various sites (Fig. 23-1 to Fig. 23-4).

Thus, these results suggest that an antibody library containing an antibody against an arbitrary antigen can be constructed by turning the mutation capacity of DT40-SW-hg ON and culturing DT40-SW-hg for a given time. These results guarantee that DT40-SW-hg is a useful cell line for antibody preparation.

All publications, patents, and patent applications cited herein are incorporated herein by reference in their entirety.

## Claims

1. A method for preparing B cells which produce a human-type antibody, wherein the B cells are derived from a DT40 chicken B cell line capable of inducing or halting AID (activation induced cytidine deaminase) expression with the induction of the expression of an exogenous Cre recombinase gene through extracellular stimulation followed by the inversion of the direction of the exogenous AID gene by expressed Cre recombinase,
the B cells are **characterized in that**:
1) the endogenous AID gene is functionally disrupted, and an AID protein resulting from the expression of the endogenous AID gene is not produced;
2) the B cells have an exogenous AID gene that is flanked by two loxP sequences in opposite directions to each other, and a promoter that is present upstream of the region flanked by the two loxP sequences and is capable of functioning in the animal cells, when the AID gene is placed in a forward direction with respect to the promoter, the AID gene can be expressed by the promoter, and
when the AID gene is placed in a reverse direction with respect to the promoter, the expression of the AID gene is halted; and
3) a Cre recombinase gene is introduced so that Cre recombinase activation by extracellular stimulation is possible, and the direction of the region flanked by the two loxP sequences, which contains the exogenous AID gene, is inverted by Cre recombinase activation,
and
the B cells are further **characterized in that**:
(i) only a constant region of an antibody gene of the B cells is substituted with a human antibody constant region gene;
(ii) in the case of the heavy chain, the region from the CH1 region to a secretory exon encoding the constant region is substituted with a human-derived IgG antibody heavy chain constant region, and in the case of the light chain, only the constant region exon is substituted with a human-derived κ light chain constant region gene; and
(iii) B cells producing a human-type antibody contain a splicing receptor sequence and a splicing branch point sequence, which are derived from a human antibody gene, in an intron that is present upstream of the human constant region gene, further contain as a polyA addition sequence downstream of the constant region gene, a polyA addition sequence of DT40-SW cells that are B cells derived from the DT40 chicken B cell line, and further contain a drug resistance gene flanked by loxP sequences upstream of the human constant region gene containing a splicing sequence.

2. The method for preparing B cells which produces a human-type antibody according to claim 1, wherein the Cre recombinase gene of B cells derived from the DT40 chicken B cell line is present in a form such that a fusion protein with an estrogen receptor is expressed, and the extracellular stimulation is carried out by estrogen or a derivative thereof, such that cells are stimulated extracellularly by estrogen or a derivative thereof, so as to induce Cre recombinase activation intracellularly.

3. The method for preparing B cells which produces a human-type antibody according to claim 1 or 2, wherein the antibody gene of B cells derived from the DT40 chicken B cell line is substituted with a human antibody gene using a targeting vector targeting the antibody gene of the B cells.

4. A method for constructing a mutant human-type antibody library, comprising activating a Cre recombinase gene in B cells derived from the DT40 chicken B cell line producing the human-type antibody prepared by the method of any one of claims 1 to 3, culturing B cells, and then introducing a mutation into the antibody variable region gene.

5. The method for constructing a mutant human-type antibody library according to claim 4, wherein Cre recombinase is present in B cells derived from the DT40 chicken B cell line producing a human-type antibody, such that a fusion protein with an estrogen receptor is expressed, Cre recombinase activation is induced intracellularly by stimulation of cells with estrogen or a derivative thereof extracellularly, and thus a mutation is introduced into the antibody variable region gene.

## Patentansprüche

1. Verfahren zum Herstellen von B-Zellen, die einen Antikörper vom Humantyp produzieren, wobei
die B-Zellen von einer DT40 Hühner-B-Zelllinie abgeleitet sind, die fähig ist, die Expression von AID (aktivierungsinduzierter Cytidindesaminase) mit der Induktion der Expression eines exogenen Cre-Rekombinase-Gens durch extrazelluläre Stimulation,
gefolgt von der Inversion der Richtung des exogenen AID-Gens durch exprimierte Cre-Rekombinase, zu induzieren oder anzuhalten,
die B-Zellen **dadurch gekennzeichnet sind, dass**:
1) das endogene AID-Gen funktionell zerstört ist und ein AID-Protein, das sich aus der Expression des endogenen AID-Gens ergibt, nicht produziert wird;
2) die B-Zellen ein exogenes AID-Gen, das durch zwei loxP-Sequenzen in entgegengesetzten Richtungen zueinander flankiert ist, und einen Promotor, der stromaufwärts der durch die beiden loxP-Sequenzen flankierten Region vorliegt und fähig ist, in den tierischen Zellen zu funktionieren, aufweisen, wobei das AID-Gen durch den Promotor exprimiert werden kann, wenn das AID-Gen in einer vorwärts gerichteten Richtung in Bezug auf den Promotor platziert wird, und die Expression des AID-Gens angehalten wird, wenn das AID-Gen in einer rückwärts gerichteten Richtung in Bezug auf den Promotor platziert wird, und
3) ein Cre-Rekombinase-Gen eingeführt wird, so dass eine Aktivierung der Cre-Rekombinase durch extrazelluläre Stimulation möglich ist und die Richtung der durch die beiden loxP-Sequenzen flankierten Region, die das exogene AID-Gen enthält, durch Aktivierung der Cre-Rekombinase umgekehrt wird, und
die B-Zellen ferner **dadurch gekennzeichnet sind, dass**:
(i) nur eine konstante Region eines Antikörpergens der B-Zellen durch ein Gen für eine humane konstante Antikörperregion ersetzt ist;
(ii) im Falle der schweren Kette die Region von der CH1-Region bis zu einem sekretorischen Exon, welche die konstante Region kodiert, durch eine konstante Region der schweren Kette eines von der humanen Sequenz abgeleiteten IgG-Antikörpers ersetzt ist und im Falle der leichten Kette nur das Exon für die konstante Region durch ein Gen für die konstante Region einer von der humanen Sequenz abgeleiteten κ-Kette ersetzt ist, und
(iii) B-Zellen, die einen Antikörper vom Humantyp produzieren, eine Spleiß-Akzeptor-Sequenz und eine Spleiß-Verzweigungs-Sequenz, die von einem humanen Antikörpergen abgeleitet sind, in einem Intron enthalten, das stromaufwärts des Gens für die humane konstante Region vorliegt, ferner
als Polyadenylierungssequenz stromabwärts des Gens für die konstante Region eine Polyadenylierungssequenz von DT40-SW-Zellen, bei denen es sich um von der DT40-Hühner-B-Zelllinie abgeleitete B-Zellen handelt, enthalten und ferner ein durch loxP-Sequenzen flankiertes Arzneimittelresistenzgen stromaufwärts des Gens für die humane konstante Region, das eine Spleißsequenz enthält, enthalten.

2. Verfahren zum Herstellen von B-Zellen, die einen Antikörper vom Humantyp produzieren, gemäß Anspruch 1, wobei das Cre-Rekombinase-Gen von B-Zellen, die von der DT40-Hühner-B-Zelllinie abgeleitet sind, in einer solchen Form vorliegt, dass ein Fusionsprotein mit einem Östrogenrezeptor exprimiert wird, und die extrazelluläre Stimulation durch Östrogen oder ein Derivat davon ausgeführt wird, so dass Zellen extrazellulär durch Östrogen oder ein Derivat davon stimuliert werden, um so eine Aktivierung der Cre-Rekombinase intrazellulär zu induzieren.

3. Verfahren zum Herstellen von B-Zellen, die einen Antikörper vom Humantyp produzieren, gemäß Anspruch 1 oder 2, wobei das Antikörpergen von B-Zellen, die von der DT40-Hühner-B-Zelllinie abgeleitet sind, unter Verwendung eines Targetingvektors, der auf das Antikörpergen der B-Zellen zielt, durch ein humanes Antikörpergen ersetzt ist.

4. Verfahren zum Konstruieren einer Bibliothek mutanter Antikörper vom Humantyp, umfassend Aktivieren eines Cre-Rekombinase-Gens in von der DT40-Hühner-B-Zelllinie abgeleiteten B-Zellen, die den Antikörper vom Humantyp produzieren, hergestellt durch das Verfahren nach einem der Ansprüche 1 bis 3, Züchten der B-Zellen und dann Induzieren einer Mutation in dem Gen für die variable Antikörperregion.

5. Verfahren zum Konstruieren einer Bibliothek mutanter Antikörper vom Humantyp gemäß Anspruch 4, wobei Cre-Rekombinase in von der DT40-Hühner-B-Zelllinie abgeleiteten B-Zellen, die einen Antikörper vom Humantyp produzieren, vorliegt, so dass ein Fusionsprotein mit einem Östrogenrezeptor exprimiert wird, eine Aktivierung der Cre-Rekombinase intrazellulär durch extrazelluläre Stimulation von Zellen mit Östrogen oder einem Derivat davon induziert wird und so eine Mutation in dem Gen für die variable Antikörperregion eingeführt wird.

## Revendications

1. Procédé de préparation de cellules B qui produisent un anticorps de type humain, où
les cellules B sont dérivées d'une lignée de cellules B de volaille DT40 capables d'induire ou de stopper l'expression de l'AID (activation induite par la cytidine désaminase) avec l'induction de l'expression d'un gène exogène de Cre recombinase à travers la stimulation extracellulaire suivie de l'inversion du sens du gène exogène AID par la Cre recombinase exprimée,
les cellules B sont **caractérisées en ce que** :
1) le gène AID endogène est fonctionnellement brisé, et une protéine AID résultant de l'expression du gène AID endogène n'est pas produite ;
2) les cellules B ont un gène AID exogène qui est flanqué de deux séquences loxP en sens opposés l'une par rapport à l'autre, et un promoteur qui est présent en amont de la région flanquée par les deux séquences loxP et est capable de fonctionner dans les cellules animales,
lorsque le gène AID est placé dans un sens avant par rapport au promoteur, le gène AID peut être exprimé par le promoteur, et
lorsque le gène AID est placé dans un sens inverse par rapport au promoteur, l'expression du gène AID est stoppée ; et
3) un gène Cre recombinase est introduit de sorte que l'activation Cre recombinase par stimulation extracellulaire est possible, et le sens de la région flanquée par les deux séquences loxP, qui contient le gène AID exogène, est inversé par l'activation Cre recombinase, et
les cellules B sont en outre **caractérisées en ce que** :
(i) uniquement une région constante d'un gène d'anticorps des cellules B soit substituée par un gène de région constante d'anticorps humain ;
(ii) dans le cas de la chaîne lourde, la région à partir de la région CH1 d'un exon sécréteur codant pour la région constante est substituée par une région constante de chaîne lourde d'anticorps IgG dérivé de l'être humain, et dans le cas de la chaîne légère, seul l'exon de région constante est substitué par un gène de région constante de chaîne légère κ dérivé de l'être humain ; et
(iii) les cellules B produisant un anticorps de type humain contient une séquence réceptrice d'épissage et une séquence de point de ramification d'épissage, qui sont dérivées d'un gène d'anticorps humain, dans un intron qui est présent en amont du gène de région constante humain, contiennent en outre,
comme séquence d'addition polyA en aval du gène de région constante, une séquence d'addition polyA de cellules DT40-SW qui sont des cellules B dérivées de la lignée de cellules B de volaille DT40, et contient en outre un gène de résistance médicamenteuse flanqué par des séquences loxP en amont du gène de région constante humain contenant une séquence d'épissage.

2. Procédé de préparation de cellules B qui produisent un anticorps type humain selon la revendication 1, où le gène de Cre recombinase des cellules B dérivées de la lignée de cellules B de volaille DT40 est présent sous une forme telle qu'une protéine de fusion avec un récepteur d'oestrogène est exprimée, et la stimulation extracellulaire est conduite par l'oestrogène ou un dérivé de celui-ci de sorte que les cellules soient stimulées de manière extracellulaire par l'oestrogène ou un dérivé de celui-ci, afin d'induire de manière intracellulaire l'activation de la Cre recombinase.

3. Procédé de préparation de cellules B qui produisent un anticorps de type humain selon la revendication 1 ou 2, où le gène d'anticorps des cellules B dérivées de la lignée de cellules B de volaille DT40 est substitué par un gène d'anticorps humain en utilisant un vecteur de ciblage ciblant le gène d'anticorps des cellules B.

4. Procédé de construction d'une bibliothèque d'anticorps mutants de type humain, comprenant l'activation d'un gène de Cre recombinase dans des cellules B dérivées de la lignée de cellules B de volaille DT40 produisant l'anticorps de type humain préparé par le procédé selon l'une quelconque des revendications 1 à 3, la culture des cellules B, et ensuite l'introduction d'une mutation dans le gène de région variable d'anticorps.

5. Procédé de construction d'une bibliothèque d'anticorps mutants de type humain selon la revendication 4, où la Cre recombinase est présente dans les cellules B dérivées de la lignée de cellules B de volaille DT40 produisant un anticorps de type humain, de sorte qu'une protéine de fusion avec un récepteur d'oestrogène soit exprimée, l'activation Cre recombinase est induite de manière intracellulaire par la stimulation des cellules avec l'oestrogène ou un dérivé de celui-ci de manière extracellulaire, et ainsi une mutation est introduite dans le gène de région variable d'anticorps.
